(19) **Europäisches Patentamt**
**European Patent Office**
**Office européen des brevets**

(11) **EP 3 870 193 B1**

(12) **EUROPEAN PATENT SPECIFICATION**

(45) Date of publication and mention
of the grant of the patent:
**08.10.2025 Bulletin 2025/41**

(21) Application number: **19876745.1**

(22) Date of filing: **24.10.2019**

(51) International Patent Classification (IPC):
***G16B 15/20*** (2019.01)    ***G16B 40/10*** (2019.01)
***G16B 40/20*** (2019.01)    *C07K 16/00* (2006.01)

(52) Cooperative Patent Classification (CPC):
**C07K 16/00;** C07K 2317/94; G16B 15/20;
G16B 40/10; G16B 40/20

(86) International application number:
**PCT/US2019/057856**

(87) International publication number:
**WO 2020/086845 (30.04.2020 Gazette 2020/18)**

(54) **SYSTEM AND METHOD FOR DETERMINING DEAMIDATION AND IMMUNOGENICITY OF POLYPEPTIDES**

SYSTEM UND VERFAHREN ZUR BESTIMMUNG DER DEAMIDIERUNG UND IMMUNOGENIZITÄT VON POLYPEPTIDEN

SYSTÈME ET PROCÉDÉ DE DÉTERMINATION DE LA DÉSAMIDATION ET DE L'IMMUNOGÉNICITÉ DE POLYPEPTIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2018 US 201862750022 P**

(43) Date of publication of application:
**01.09.2021 Bulletin 2021/35**

(73) Proprietor: **Protein Dynamic Solutions, Inc. Wakefield, MA 01880 (US)**

(72) Inventors:
• **PASTRANA-RIOS, Belinda**
**Wakefield, Massachusetts 01880 (US)**
• **NODA, Isao**
**Fairfield, Ohio 45014 (US)**

(74) Representative: **V.O.**
**P.O. Box 87930**
**2508 DH Den Haag (NL)**

(56) References cited:
WO-A1-2017/127679    WO-A1-2017/127679
WO-A1-2018/231840    US-B1- 8 268 628

• HÁDA VIKTOR ET AL: "Recent advancements, challenges, and practical considerations in the mass spectrometry-based analytics of protein biotherapeutics: A viewpoint from the biosimilar industry", JOURNAL OF PHARMACEUTICAL AND BIOMEDICAL ANALYSIS, ELSEVIER B.V, AMSTERDAM, NL, vol. 161, 11 August 2018 (2018-08-11), pages 214 - 238, XP085493898, ISSN: 0731-7085, DOI: 10.1016/ J.JPBA.2018.08.024
• NODA ISAO: "Two-dimensional codistribution spectroscopy to determine the sequential order of distributed presence of species", JOURNAL OF MOLECULAR STRUCTURE, vol. 1069, 24 January 2014 (2014-01-24), pages 50 - 59, XP029028456, ISSN: 0022-2860, DOI: 10.1016/ J.MOLSTRUC.2014.01.024
• ANONYMOUS: "Two-dimensional correlation analysis", WIKIPEDIA, 18 August 2018 (2018-08-18), pages 1 - 6, XP055799310

EP 3 870 193 B1

**Description**

Background

High attrition rates of drug candidates, such as protein therapeutics, is the main costs driver in drug development and continues to be a key challenge in the biopharmaceutical industry. Immunogenicity, protein aggregation, deamidation, and oxidation are of concern to regulatory agencies due to the impact they may have in decreased efficacy and safety for the patients. Proteins are complex molecules that are exposed to the potential of non-enzymatic deamidation of asparagine conversion to aspartate or glutamine to glutamate under varying conditions. The occurrence of the isomer product is observed only at high pH conditions. Specifically, the process of deamidation in proteins has been associated with both low and high pH conditions, as well as thermal stress. Therefore, the risk of occurrence includes: upstream processing during the: (1) cell culture production of the therapeutic protein, and/or downstream processing during: (2) purification, (3) viral clearance and during storage and delivery and (4) thermal stress and/or low/high pH condition.

There are currently limitations to evaluating deamidation for proteins in solution in a high-throughput manner. Current techniques, such as HPLC, NMR and MS, have limitations regarding the number of samples that can be analyzed, the assessment of the stability of the protein as a result of the deamidation and the resulting effects on efficacy and safety.

The mechanism of deamidation is kinetically driven, and requires the neighboring residue (N+1) to be small to prevent stearic hindrance; allowing for the succinimide intermediate to be formed which follows the hydrolysis of the $-NH_2$ group resulting in the negatively charged residue. The current technology used to detect deamidation is based on separation of charge variants by high performance liquid chromatography ("HPLC") such as ion exchange (IEX) or reverse phase. Then nuclear magnetic resonance spectroscopy ("NMR") is used to identify structural and primary sequence changes within the protein. Mass spectrometry ("MS") has also been developed for asparagine deamidation detection of the isoaspartate only at high pH. The MS technique requires the fragmentation of the full-length charge variant protein, and peptide mapping for the exclusive detection of the isoaspartate mass difference. This is a complex and time consuming process. As an example, HÁDA VIKTOR ET AL: "Recent advancements, challenges, and practical considerations in the mass spectrometry-based analytics of protein biotherapeutics: A viewpoint from the biosimilar industry",JOURNAL OF PHARMACEUTICAL AND BIOMEDICAL ANALYSIS, ELSEVIER B.V, AMSTERDAM, NL, vol. 161, 11 August 2018 (2018-08-11), pages 214-238 discloses the analysis of deamidation of amino acid site chains by MS.

Summary of the Invention

The subject technology is illustrated, for example, according to various aspects described below. Various examples of aspects of the subject technology are described below. These are provided as examples and do not limit the subject technology.

Aspects of the subject technology provide a system and method for determining and assessing deamidation of protein samples under thermal duress. In particular, the system and methods provide for determining assessing deamidation within glutamines and asparagines, for the determination of aggregate size, identity, extent and mechanism of aggregation, as well as stability, target binding and the validation of bioassays. The system and methods allow for developability and comparability assessment of therapeutic proteins independent of their molecular weight, post-translational modification and/or formulation condition with only 1 μL volumes per sample. Furthermore, the empirical results can directly impact protein design and re-engineering.

According to one aspect of the subject technology, the system and methods described herein involve obtaining and analyzing spectral data for proteins, including infrared (IR) spectra, such as IR spectra obtained using a quantum cascade laser ("QCL") microscope. The system and methods provide real-time high-throughput hyperspectral imaging ("HSI") that allows for the monitoring of an array of proteins in solution during thermal stress. Unlike certain existing methods of monitoring proteins, the method does not require a separation technique, and it does not comprise a flow channel. The system uses a QCL transmission microscope with linear response detection based on first principle, accurate thermal control, and unique heated cell holder with a multiplexed array slide cell that allows for fixed volume requirements. This provides a fast acquisition system, up to 200 times faster than Fourier transform infrared ("FT-IR") microscopes, with enhanced signal to noise ratio ("SNR") capable of determining the size, identity, extent and mechanism of aggregation. The QCL microscope spectral data is processed using analytical algorithms, as described herein, to determine the existence and extent of deamidation. The system and method can also process the spectral data to monitor and assess colloidal stability, or evaluate other stressor conditions such as pH.

The system and method provides for the analysis of hundreds of samples a day. The methods employed in the spectral analysis are used to map the regions of deamidation, identify the regions prone to aggregation, and establish domain stability. Correlation dynamics software included in the system, and used to implement aspects of the methods, allows for the correlation of side chain modes which are used to probe the protein in solution under the stressor condition. As a result the data is highly informative and statistically robust.

According to one aspect of the technology, systems and methods use HSI for the real-time monitoring and analysis of the event of deamidation of proteins under thermal and/or chemical stress, including using an array of therapeutic proteins in solution. The results of such monitoring and analysis have predictive implications, while allowing for the mapping of the site that is prone to deamidation. For example, deamidation can be predictive of immunogenicity and/or a tendency to aggregate. The results are statistically robust. Furthermore, by analyzing variants of the protein candidate, a comprehensive body of evidence can be provided for pre-clinical candidate selection early in discovery phase. Moreover, the subject technology is also capable of describing protein aggregation mechanism and unfolding, thereby providing molecular detail of the events that can lead to immunogenicity. Therapeutic protein candidate selection is based on the predictive power of the data processing and analysis methods described herein, based on HIS acquired using a QCL microscope. Other analytical tools currently used to assess deamidation occurrence, including HPLC, NMR and MS, can also be used in combination with the system and methods disclosed herein, thus allowing the selection of a stable candidate resulting in lower risk of candidate withdrawal, while ensuring efficacy and safety.

The methods, systems, and instructions for processing data described herein can be used to assess deamidation, aggregation and the potential for immunogenicity as part of the development of protein therapeutics. Studies performed on protein samples using the subject technology demonstrate the assessment and determination of deamidation of asparagine and/or glutamine residues in an array of proteins in solution, and provide a validation of immunogenicity and anti-drug antibody (ADA) bioassays by providing a direct method of detection of drug substance ("DS") and/or drug product ("DP") aggregation in vitro or in situ.

Additional features and advantages of the subject technology will be set forth in the description below, and in part will be apparent from the description, or may be learned by practice of the subject technology. The advantages of the subject technology will be realized and attained by the structure particularly pointed out in the written description and claims hereof as well as the appended drawings.

It is to be understood that both the foregoing general description and the following detailed description are exemplary and explanatory and are intended to provide further explanation of the subject technology as claimed.

Brief Description of the Drawings

The accompanying drawings, which are included to provide further understanding of the subject technology and are incorporated in and constitute a part of this description, illustrate aspects of the subject technology and, together with the specification, serve to explain principles of the subject technology.

FIG. 1 shows a diagram of an exemplary computing system according to some aspects of the subject technology.

FIG. 2A shows a flowchart indicating operations of an exemplary method verifying and preparing input data, according to some aspects of the subject technology.

FIG. 2B shows a flowchart indicating operations of an exemplary method according to some aspects of the subject technology.

FIG. 3 shows results of a multi-stage analysis.

FIG. 4A shows hyperspectral images (HSI) generated using a low magnification objective with a field of view of 2 x 2 $mm^2$ for PDS NIST mAb RM 8671 at 1 $\mu g/\mu L$, at 28°C and 56°C.

FIG. 4B shows hyperspectral images (HSI) generated using a low magnification objective with a field of view of 2 x 2 $mm^2$ for NIST mAb RM 8671 at 2 $\mu g/\mu L$, at 28°C and 56°C.

FIG. 4C shows hyperspectral images (HSI) generated using a low magnification objective with a field of view of 2 x 2 $mm^2$ for P NIST mAb Candidate RM 8670 at 2.4 $\mu g/\mu L$, at 28°C and 56°C.

FIG. 5 shows QCL IR spectral overlays of the amide I and II bands with overlapping L-Histidine and $H_2O$ absorption in the spectral region of 1780 - 1450 $cm^{-1}$ within the temperature range of 28-56 °C with 4°C temperature intervals: 28°C, 32°C, 36 °C, 40 °C, 44 °C, 48 °C, 52 °C, 56°C.

FIG. 6A shows the QCL spectral overlay of amide I and amide II bands within the spectral region of 1780 - 1450 cm-1 corresponding to the temperature range of 28-56 °C for PDS NIST mAb at 1 $\mu g/\mu L$.

FIG. 6B shows the synchronous plot generated based on the QCL spectral overlay data shown in FIG. 6A.

FIG. 6C shows the asynchronous plot generated based on the QCL spectral overlay data shown in FIG. 6A.

FIG. 7A shows the QCL spectral overlay of amide I and amide II bands within the spectral region of 1780 - 1450 $cm^{-1}$ corresponding to the temperature range of 28-56 °C for NIST mAb at 1 $\mu g/\mu L$.

FIG. 7B shows the synchronous plot generated based on the QCL spectral overlay data shown in FIG. 7A.

FIG. 7C shows the asynchronous plot generated based on the QCL spectral overlay data shown in FIG. 7A.

FIG. 8A shows the QCL spectral overlay of amide I and amide II bands within the spectral region of 1780 - 1450 $cm^{-1}$ corresponding to the temperature range of 28-56 °C for NIST mAb Candidate at 1.5 $\mu g/\mu L$.

FIG. 8B shows the synchronous plot generated based on the QCL spectral overlay data shown in FIG. 8A.

FIG. 8C shows the asynchronous plot generated based on the QCL spectral overlay data shown in FIG. 8A.

FIG. 9A shows the sequential order of events for PDS NIST mAb at 1 μg/μL thermally stressed within the temperature range of 28-56 °C.

FIG. 9B shows the sequential order of events for NIST mAb at 1 μg/μL thermally stressed within the temperature range of 28-56 °C.

FIG. 9C shows the sequential order of events for NIST mAb Candidate at 1.5 μg/μL thermally stressed within the temperature range of 28-56 °C.

FIG. 10A shows the QCL spectral overlay of amide I and amide II bands within the spectral region of 1780 - 1450 cm-1 corresponding to the temperature range of 28-56 °C for PDS NIST mAb at 2 μg/μL.

FIG. 10B shows the synchronous plot generated based on the QCL spectral overlay data shown in FIG. 10A.

FIG. 10C shows the asynchronous plot generated based on the QCL spectral overlay data shown in FIG. 10A.

FIG. 11A shows the QCL spectral overlay of amide I and amide II bands within the spectral region of 1780 - 1450 cm$^{-1}$ corresponding to the temperature range of 28-56 °C for NIST mAb at 2 μg/μL.

FIG. 11B shows the synchronous plot generated based on the QCL spectral overlay data shown in FIG. 11A.

FIG. 11C shows the asynchronous plot generated based on the QCL spectral overlay data shown in FIG. 11A.

FIG. 12A shows the QCL spectral overlay of amide I and amide II bands within the spectral region of 1780 - 1450 cm$^{-1}$ corresponding to the temperature range of 28-56 °C for NIST mAb Candidate at 2.4 μg/μL.

FIG. 12B shows the synchronous plot generated based on the QCL spectral overlay data shown in FIG. 12A.

FIG. 12C shows the asynchronous plot generated based on the QCL spectral overlay data shown in FIG. 12A.

FIG. 13A shows the sequential order of events for PDS NIST mAb at 2 μg/μL thermally stressed within the temperature range of 28-56 °C.

FIG. 13B shows the sequential order of events for NIST mAb at 2 μg/μL thermally stressed within the temperature range of 28-56 °C.

FIG. 13C shows the sequential order of events for NIST mAb Candidate at 2.4 μg/μL thermally stressed within the temperature range of 28-56 °C.

FIG. 14A shows the QCL spectral overlay of amide I and amide II bands within the spectral region of 1780 - 1450 cm$^{-1}$ corresponding to the temperature range of 28-56 °C for the NIST mAb sample at 2.8 μg/μL.

FIG. 14B shows the synchronous plot generated based on the QCL spectral overlay data shown in FIG. 14A.

FIG. 14C shows the asynchronous plot generated based on the QCL spectral overlay data shown in FIG. 14A.

FIG. 15A shows the QCL spectral overlay of amide I and amide II bands within the spectral region of 1780 - 1450 cm$^{-1}$ corresponding to the temperature range of 28-56 °C for NIST mAb Candidate at 10.0 μg/μL.

FIG. 15B shows the synchronous plot generated based on the QCL spectral overlay data shown in FIG. 15A.

FIG. 15C shows the asynchronous plot generated based on the QCL spectral overlay data shown in FIG. 15A.

FIG. 16A shows the sequential order of events for PDS NIST mAb at 2.8 μg/μL thermally stressed within the temperature range of 28-56 °C.

FIG. 16B shows the sequential order of events for NIST mAb Candidate at 10.0 μg/μL thermally stressed within the temperature range of 28-56 °C.

FIGS. 17A, 17B and 17C are asynchronous plots for PDS NIST mAb standard (RM 8671), NIST mAb standard (RM 8671), and NIST mAb candidate (RM 8670) at low concentration demonstrating evidence of deamidation.

FIGS. 18A, 18B and 18C are bar graphs illustrating intensity changes within cross peaks associated with deamidation.

FIG. 19 is an asynchronous plot for NIST mAb Candidate at low concentration during thermal stress demonstrating evidence of deamidation.

FIG. 20 is a bar graph illustrating intensity changes within cross peaks associated with deamidation.

FIG. 21 is a schematic representation of the mechanism of deamidation for asparagine along with key vibrational modes that are used to monitor the event during thermal stress.

FIG. 22 is an illustration of exemplary platform technology that may be used to implement the systems and methods of the subject technology.

FIG. 23 is a flow chart indicating operations of an exemplary design of experiments method according to some aspects of the subject technology.

FIG. 24 is a flow chart indicating operations of exemplary methods for ADA screening and immunogenicity risk assessment.

FIG. 25 is a flow chart indicating operations of an exemplary comparative analysis that may be performed using the platform technology and methods described herein.

FIG. 26 shows an exemplary diagram of a computing system.

Detailed Description of the Invention

In the following detailed description, specific details are set forth to provide an understanding of the subject technology. It will be apparent, however, to one ordinarily skilled in the art that the subject technology may be practiced without some of these specific details. In other instances, well-known structures and techniques have not been shown in detail so as not to obscure the subject technology.

Proteins are large organic compounds made of amino acids arranged in a linear chain and joined together by peptide bonds between the carboxyl and amino groups of adjacent amino acid residues. Most proteins fold into unique 3-dimensional structures. The shape into which a protein naturally folds is known as its native state. Although many proteins can fold unassisted, simply through the chemical properties of their amino acids, others require the aid of molecular chaperones to fold into their native states. There are four distinct aspects of a protein's structure:

- Primary structure: the amino acid sequence.
- Secondary structure: regularly repeating local structures stabilized by hydrogen bonds. Because secondary structures are local, many regions of different secondary structure can be present in the same protein molecule.
- Tertiary structure: the overall shape of a single protein molecule; the spatial relationship of the secondary structures to one another.
- Quaternary structure: the shape or structure that results from the interaction of more than one protein molecule, usually called protein subunits in this context, which function as part of the larger assembly or protein complex.

Proteins are not entirely rigid molecules. In addition to these levels of structure, proteins may shift between several related structures while they perform their biological function. In the context of these functional rearrangements, these tertiary or quaternary structures are usually referred to as "conformations," and transitions between them are called conformational changes.

Protein aggregation is characterized as a misfolded, rigid protein grouping which is considered a prevalent phenomenon throughout the industrial bioprocess. Aggregation is considered a primary mode of protein degradation, often leading to immunogenicity of the protein and a loss of bioactivity. Protein aggregation is of critical importance in a wide variety of biomedical situations, ranging from abnormal disease states, such as Alzheimer's and Parkinson's disease, to the production, stability and delivery of protein drugs.

Deamidation is considered as a post-translational modification of proteins following protein biosynthesis that can potentially affect the stability, structure and efficacy of a therapeutic protein and may cause aggregation which can lead to an unwanted immune response such as immunogenicity and anti-drug antibody response (ADA). The residues that exhibit deamidation are asparagine and to a lesser extent glutamine. Deamidation results in the conversion of asparagine to aspartate and/or glutamine to glutamate. The negative charge introduced at the site can lead to decreased stability of the protein, causing the protein to aggregate, degradation, loose binding selectivity and affinity to its target resulting in loss of efficacy and safety. Asparagine post-translational modification occurs readily when its neighboring residue (position N+1) is glycine, lowering steric hindrance for the succinimide intermediate to form, to produce aspartate or isoaspartate. The event of deamidation occurs in the absence of any enzyme and is accelerated at high pH and/or temperature. Deamidation may signal degradation of the protein within the cell, thus decreasing the therapeutics protein half-life within the cell thus potentially affecting PK/PD.

Aspects of the subject technology provide a fast, accurate, and reproducible technique for real-time monitoring of the event of deamidation under thermal and/or chemical stress for an array of therapeutic proteins in solution. For example the subject technology provides a technique to assess and monitor asparagine and glutamine deamidation under thermal stress at high and low pH for therapeutic proteins in solution. The systems and methods described herein allow for the comparability assessment of full-length monoclonal antibodies under varying concentration and thermal stressor conditions. Comparisons real-time high-throughput HSI allow for the monitoring of the array of proteins in solution during thermal stress. Spectral data from the HSI can be analyzed to generate covariance (difference) spectra. 2D IR correlation techniques can then be applied to the covariance spectra, generating synchronous and asynchronous plots. Intensity peaks within the synchronous and asynchronous plots, along with changes in the intensities, may be analyzed. Changes in intensity and peak shifts within a spectral region of interest are analyzed, and represent the behavior of the protein under thermal stress. Correlation between peaks can be used to establish deamidation occurrence for the sample. The description of the behavior of the proteins in solution can be provided by determining the sequential order of molecular events during the thermal stress for each sample within an array. Regions where deamidation has occurred may be mapped, and the stability of the protein being examined may be determined based on the extent of deamidation and thermal stability based on the sequential order of molecular events.

The computational methods and systems described herein provide significant improvements over existing analysis for proteins. The computational methods and systems described herein generates and stores data in forms that facilitate efficient and meaningful analysis without requiring the use of several pieces of equipment. Accordingly, the computational methods and systems described herein can improve the efficiency of spectral data analysis for evaluation of candidate drugs.

Aspects of the subject technology include the use of two-dimensional correlation spectroscopy ("2DCOS") and/or two-dimensional co-distribution spectroscopy ("2DCDS") to provide essential information towards the extent and mechanism of deamidation of a protein therapeutic. The methods described herein can include analysis of the side chain modes as internal probes, offering information that confirms the stability of the structural motif or domain within proteins. The methods described herein have been shown to be useful in High Throughput-Developability and Comparability Assessment ("HT-DCA") via a Design of Experiment ("DOE") approach that complied with Quality by Design ("QBD").

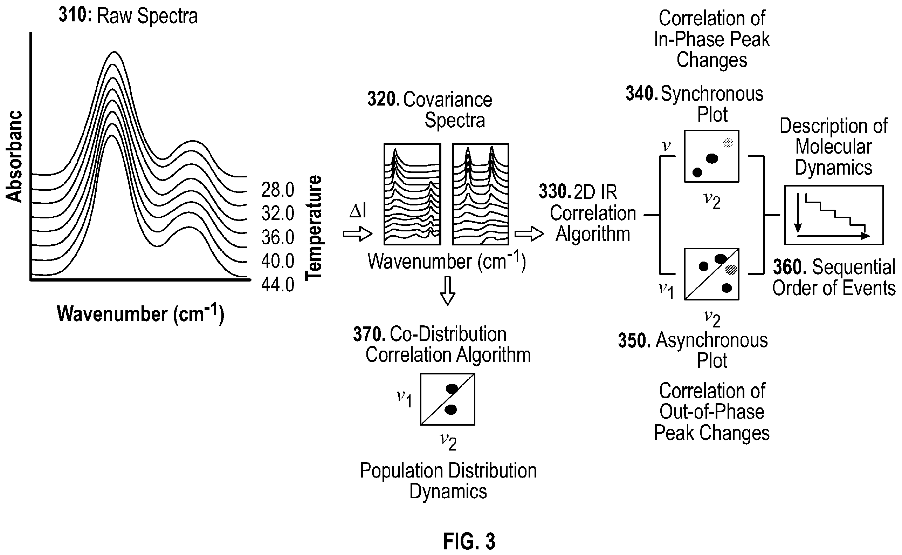

According to some embodiments, spectral analysis can be performed in stages, for example as illustrated in FIG. 3. According to some embodiments, the protein in solution sample is perturbed (thermally, chemically, pressure, or acoustics) inducing a dynamic fluctuation in the vibrational spectrum. In stage 310, raw spectra data can be collected and/or analyzed. The spectral data can be acquired at regular temperature intervals and in a sequential manner. According to some embodiments, the data can be baseline corrected.

According to some embodiments, the spectral data can be used to determine the existence and extent of deamidation events. For this, the first, low temperature mean spectrum is subtracted from the subsequent spectra to generate the dynamic spectra. In stage 320, covariance (difference) spectra can be generated by subtraction of the first, low temperature mean spectrum (24°C) from all subsequent spectra. Consequently, the covariance (difference) spectra contain positive and negative peaks; also referred as in- and out-of-phase from one another.

Notably the process described herein does not require the manual subtraction of water or other reference (e.g., solute) from spectral data. Such manual subtraction is a highly subjective step often incurred in protein spectral analysis. Instead, the process described herein generates the difference spectral data set based on the perturbation of the sample of interest. The output thereof can then be used for further analysis. By subtracting the first, low temperature mean spectrum which has the overlapping water band along with the amide I band from all subsequent spectra, the spectral contributions of water are automatically subtracted. That is, the contribution of water and all protein vibrational modes that were not perturbed, such as by thermal stress, were subtracted, allowing for the evaluation of only the changes that occurred in the spectral region of interest ($1780 - 1450$ cm$^{-1}$) upon thermal stress.

The detailed molecular evaluation of the protein in solution is then obtained by applying a 2D IR correlation technique, as shown in stage 330. In stage 330, the 2D IR correlation technique can be applied to generate a synchronous plot (stage 340) and an asynchronous plot (stage 350). For example, the spectral data can be fast Fourier transformed ("FFT") to generate the complex matrix from which an intensity matrix is obtained through the cross correlation product the synchronous and asynchronous plots are generated.

The synchronous plot represents the overall intensity changes that occur during the perturbation within the spectral region of interest. On the diagonal of this plot are the peaks or bands (known as auto peaks) that changed throughout the spectrum. Off the diagonal are the cross peaks which show the correlation between the auto peaks, that is, the relationship between the secondary structure changes observed. The synchronous plot can be used to relate the in-phase peak intensity changes or shifts.

In synchronous correlation spectrum, auto peaks at diagonal positions represent the extent of perturbation-induced dynamic fluctuations of spectral signals. Cross peaks represent simultaneous changes of spectral signals at two different wavenumbers, suggesting a coupled or related origin of intensity variations. If the sign of a cross peak is positive, the intensities at corresponding wavenumbers are increasing or decreasing together. If the sign is negative, one is increasing, while the other is decreasing.

The asynchronous plot contains only cross peaks which are used to determine the sequential order of molecular events that occurred as a function of the thermal stress or other applied perturbation. The asynchronous plot can be used to relate the out-of-phase peak intensity changes or shifts that occurred as a function of the thermal stress. For example, observation of decreased intensity for asparagine at 1612.7 cm$^{-1}$ associated $\delta(NH_2)$ vibrational mode, along with an observed concomitant increase in intensity for the aspartate intensity at 1572.0 cm$^{-1}$ $\nu(COO^-)$ vibrational mode can be used to indicate a deamidation.

In the asynchronous correlation spectrum, cross peaks develop only if the intensity varies out of phase with each other for some Fourier frequency components of signal fluctuations. The sign of a cross peak is positive if the intensity change at wavenumber $\nu_2$ occurs before wavenumber $\nu_1$. The sign of a cross peak is negative if the intensity change at wavenumber $\nu_2$ occurs after wavenumber $\nu_1$. The above sign rules are reversed if the same asynchronous cross peak position translated to the synchronous plot falls in a negative region ($\Phi(\nu_1, \nu_2) < 0$).

The 2D IR correlation spectroscopy can be used to resolve the complex bands, such as the amide I and II bands. In particular, 2D IR correlation enhances the spectral resolution of the underlying peaks of broad bands such as the amide I and II bands by spreading the peaks in two dimensions. As mentioned, the 2D IR correlation technique generates a synchronous plot and an asynchronous plot. These plots are symmetrical in nature, and for discussion purposes reference will be made to the top triangle for analysis. The synchronous plot (shown at 340) contains two types of peaks: (a) auto peaks that are positive peaks on the diagonal and (b) cross peaks that are off-diagonal peaks that can be either positive or negative. The asynchronous plot (shown at 350) is comprised exclusively of cross peaks that relate the out-of-phase peaks. As a result this plot reveals greater spectral resolution enhancement. The following rules can apply to establish the order of molecular events:

I. If the asynchronous cross peak, $v_2$, is positive, then $v_2$ *is* perturbed prior to $v_1$ ($v_2 \rightarrow v_1$).

II. If the asynchronous cross peak, $v_2$, is negative, then $v_2$ is perturbed after $v_1$. ($v_2 \leftarrow v_1$).

III. If the synchronous cross peak (off-diagonal peaks, not shown in FIG. 3) are positive, then the order of events are exclusively established using the asynchronous plot (rules I and II).

IV. If the synchronous plot contains negative cross peaks and the corresponding asynchronous cross peak is positive, then the order is reversed.

V. If the synchronous plot contains negative cross peaks and the corresponding asynchronous cross peak is negative, then the order is maintained.

The order of events can be established for each peak observed in the $v_{2axis}$. A table can be provided summarizing the order for each event. In stage 360, a sequential order of events plot is generated using the table summarizing the order of each event. On top of each step (event) is the spectroscopic information of the cross peak, $v_2$, while on the bottom of each step is the corresponding peak assignment or the biochemical information for each event in the order in which they are perturbed as a function of temperature. Examples are provided herein.

The skilled artisan's attention is called to Dr. Isao Noda, "Two-dimensional co-distribution spectroscopy to determine the sequential order of distributed presence of species", Journal of Molecular Structure, Vol. 1069, pp. 51-54, which describes algorithms suitable for use in 2D IR correlation analysis. A summary of 2D IR correlation spectroscopy, as developed by Dr. Isao Noda, using the infrared series of sequential spectra of sample proteins is as follows. Sample proteins may include monoclonal antibodies (mAbs). For example, the use of QCL IR spectra as a function of a perturbation, in this case thermal stress (28-56°C), can be used to obtain a covariance (difference) spectral data set by subtraction of the initial spectrum from all subsequent spectra. A discretely sampled set of spectra $A(v_j, t_k)$ can be obtained for a system measured under the influence of an external perturbation, which induces changes in the observed spectral intensities. The spectral variable $v_j$ with $j = 1,2, ..., n$ may be for example wave-number, frequency, scattering angle, etc., and the other variable $t_k$ with $k = 1,2,...,m$ represents the effect of the applied perturbation, e.g., time, temperature, and electrical potential. Only the sequentially sampled spectral data set obtained during the explicitly defined observation interval between $t_1$ and $t_m$ will be used for the 2D IR correlation analysis. For simplicity, wavenumber and time are used here to designate the two variables, but it is understood that use of other physical variables is also valid.

Covariance (difference) spectra used in 2D IR correlation spectroscopy are defined as:

$$\tilde{A}(v_j, t_k) = \begin{cases} A(v_j, t_k) - \bar{A}(v_j) & for\ 1 \le k \le m \\ 0 & otherwise \end{cases} \quad (1)$$

where, $\bar{A}(v_j)$ is the initial spectrum of the data set to generate the covariance spectra. In the absence of the a priori knowledge of the reference state, the reference spectrum can also be set as the time-averaged spectrum over the observation interval between $t_1$ and $t_m$.

Synchronous 2D correlation intensities of the covariance spectral data are defined by:

$$\Phi(v_1, v_2) = \tilde{A}(v_1, t_j) \cdot \tilde{A}(v_2, t_j) \quad (2)$$

Asynchronous 2D correlation intensities of the covariance spectral data are defined by:

$$\Psi(v_1, v_2) = \tilde{A}(v_1, t_j) \cdot N_{ij}\tilde{A}(v_2, t_i) \quad (3)$$

The term $N_{ij}$ is the element of the so-called Hilbert-Noda transformation matrix, given by:

$$N_{ij} = \begin{cases} 0 & for\ i = j \\ \frac{1}{\pi(j-i)} & otherwise \end{cases} \quad (4)$$

It is to this difference spectral data set that a cross correlation function is applied, which results in two separate, yet symmetrical 2D plots. The resulting correlation intensity $\Phi(v_1, v_2)$ as a function of two independent wavenumber axes, $v_1$ and $v_2$, is the synchronous plot. The resulting correlation intensity $\psi(v_1, v_2)$ as a function of two independent wavenumbers, $v_1$ and $v_2$, is the asynchronous plot. The synchronous plot contains positive peaks on the diagonal, known as the *auto peaks*, and summarizes the changes observed in the spectral data set. The relationship established in this synchronous plot relates the spectral intensity changes that are in-phase to one another (occurring concomitantly). The asynchronous plot is a contour plot that relates the out-of-phase intensity changes, enhances the resolution of the spectral region of interest, and can easily be distinguished from the synchronous plot because it lacks peaks on the diagonal. Both plots contain off-diagonal peaks, which are referred to as *cross peaks*, these peaks correlate the spectral changes observed. Spectral intensity changes observed are due to the incremental thermal stress applied to the protein sample. Therefore, the information from both the synchronous and asynchronous plots allows for the determination of the sequential order of molecular events that occur during the stressor event or condition following Noda's rules. The synchronous and asynchronous plots are symmetrical in nature and, again, for discussion purposes we will always refer to the top triangle for analysis. To determine the sequential order of molecular events, we begin with the plot that has the greatest resolution enhancement (i. e., the asynchronous plot):

I. asynchronous *cross peak,* $v_2$ *if positive, then* $v_2$ *is perturbed prior to* $v_1$ *(*$v_2 \rightarrow v_1$*).*
II. asynchronous *cross peak,* $v_2$ *if negative then* $v_2$ *is perturbed after to* $v_1$. *(*$v_2 \leftarrow v_1$*)*
III. If the corresponding synchronous *cross peak is* positive, then the order of the event is established using the asynchronous plot (rules I and II).
IV. However, if the corresponding synchronous *cross peak* is negative and the asynchronous *cross peak* is positive then the order is reversed.

The sequential order of molecular events can be established for each peak of interest in the defined spectral region observed in the $v_2$ axis. The peaks of interests are then used in the assessment of deamidation in proteins under thermal stress, as described herein.

Referring again to FIG. XX, in stage 370, a co-distribution correlation plot provides the perturbed regions of the protein population distribution (80% threshold) in solution.

Co-distribution correlation analysis provides the common behavior of a distribution population of proteins in solution. The skilled artisan's attention is called to Isao Noda, "Two-dimensional co-distribution spectroscopy to determine the sequential order of distributed presence of species", Journal of Molecular Structure, Vol. 1069, pp. 54-56, which describes algorithms suitable for use in 2DCDS analysis.

For a set of *m* time-dependent spectra $A(v_j, t_k)$ sequentially obtained during the observation interval of $t_1 \leq t_k \leq t_m$ with the time-averaged spectrum $\overline{A}(v_j)$ given by Eq. (2), the characteristic (time) index is defined as:

$$\bar{k}(v_j) = \frac{1}{m\bar{A}(v_j)}\sum_{k=1}^{m}k \cdot A(v_j, t_k) = \frac{1}{m\bar{A}(v_j)}\sum_{k=1}^{m}k \cdot \tilde{A}(v_j, t_k) + \frac{m+1}{2} \quad (5)$$

Dynamic spectrum $\tilde{A}(v_j, t_k)$ used here is the same as that defined in Eq. (1). The corresponding characteristic time of the distribution of spectral intensity observed at wavenumber $v_j$ is given by

$$\bar{t}(v_j) = (t_m - t_1)\frac{\bar{k}(v_j) - 1}{m - 1} + t_1 \quad (6)$$

Once again, it is understood that time used here is meant to be the generic description of a representative variable of applied perturbation, so that it could be replaced with any other appropriate physical variables, such as temperature, concentration, and pressure, selected specific to the experimental condition. The characteristic time $\bar{t}(v_j)$ is the first moment (about the origin of time axis, i.e., $t = 0$) of the distribution density of the spectral intensity $A(v_j, t_k)$ along the time axis bound by the observation interval between $t_1$ and $t_m$. It corresponds to the position of the center of gravity for observed spectral intensity distributed over the time.

Given the characteristic times, $\bar{t}(v_1)$ and $\bar{t}(v_2)$, of the time distributions of spectral intensities measured at two different wave-numbers, $v_1$ and $v_2$, the synchronous and asynchronous co-distribution spectra are defined as:

$$\Gamma(v_1, v_2) = \sqrt{1 - \left(\frac{\bar{t}(v_2) - \bar{t}(v_1)}{t_m - t_1}\right)^2}\, T(v_1, v_2) \quad (7)$$

where, $T(v_1, v_2)$ is the total joint variance given by:

$$\Lambda(v_1, v_2) = \frac{\bar{t}(v_2) - \bar{t}(v_1)}{t_m - t_1}T(v_1, v_2) \quad (8)$$

$$T(v_1, v_2) = \sqrt{\Phi(v_1, v_1) \cdot \Phi(v_2, v_2)} \qquad (9)$$

Synchronous co-distribution intensity $\Gamma(v_1, v_2)$ is a measure of the coexistence or overlap of distributions of two separate spectral intensities along the time axis. In contrast, asynchronous co-distribution intensity $\Delta(v_1, v_2)$ is a measure of the difference in the distribution of two spectral signals. The term "co-distribution" denotes the comparison of two separate distributions, distinguishing this metric from the concept of "correlation" which is based on the comparison of two variations.

By combining Eqs. 5, 6, and 8, the expression for asynchronous co-distribution spectrum is given as:

$$\Delta(v_1, v_2) = \frac{T(v_1, v_2)}{m(m-1)} \sum_{k=1}^{m} k \left\{ \frac{A(v_2, t_k)}{\overline{A}(v_2)} - \frac{A(v_1, t_k)}{\overline{A}(v_1)} \right\}$$

$$= \frac{T(v_1, v_2)}{m(m-1)} \sum_{k=1}^{m} k \left\{ \frac{\tilde{A}(v_2, t_k)}{\overline{A}(v_2)} - \frac{\tilde{A}(v_1, t_k)}{\overline{A}(v_1)} \right\} \qquad (10)$$

The value of $\Delta(v_1, v_2)$ is set to be zero, if the condition of $\overline{A}(v_1) = 0$ or $\overline{A}(v_2) = 0$ is encountered, which indicates the lack of spectral intensity signals at either of the wavenumber. Synchronous co-distribution spectrum can be obtained from the relationship:

$$\Gamma(v_1, v_2) = \sqrt{T(v_1, v_1)^2 - \Delta(v_2, v_2)^2} \qquad (11)$$

In an asynchronous co-distribution spectrum, and for a cross peak with positive sign, i.e., $\Delta(v_1, v_2) = 0$, the presence of spectral intensity at $v_1$ is distributed predominantly at the earlier stage along the time axis compared to that for $v_2$. On the other hand, if $\Delta(v_1, v_2) < 0$, the order is reversed. In the case of $\Delta(v_1, v_2) \approx 0$, the average distributions of the spectral intensities observed at two wavenumbers over the time course are similar. Sign of synchronous co-distribution peaks is always positive, which somewhat limits the information content of synchronous spectrum beyond the obvious qualitative measure of the degree of overlap of distribution patterns.

Co-distribution (2DCDS) analysis is capable of providing elements of the stability of the protein, or aggregation state in a protein or any process being investigated in a weighted fashion. 2DCDS can be used to directly provide the sequence of distributed presence of species along during stress (e.g., temperature, concentration, pH, etc.) variable axis. The technique can be used as a complementary tool to augment 2DCOS analysis in directly identifying the presence of intermediate species. According to some embodiments, perturbation-dependent spectra are sequentially obtained during an observation interval. 2D correlation spectra (synchronous spectrum and asynchronous spectrum) are derived from the spectral variations. Synchronous co-distribution intensity is measured as the coexistence or overlap of distributions of two separate spectral intensities along the perturbation axis. Asynchronous co-distribution intensity is measured as the difference in the distribution of two spectral signals. For a cross peak with positive sign, i.e., $\Delta(v_1, v_2) > 0$, the presence of spectral intensity at $v_1$ is distributed predominantly at the earlier stage along the time axis compared to that for $v_2$. On the other hand, if $\Delta(v_1, v_2) < 0$, the order is reversed. In the case of $\Delta(v_1, v_2) \approx 0$, the average distributions of the spectral intensities observed at two wavenumbers over the time course are similar.

Differences between the 2DCOS analyses provide a mean average description of the pathway due to the perturbation process and its effect on the sample, while the 2DCDS analysis provides the weighted elements in a population of molecules (proteins) during the perturbation process. The result of 2DCOS and 2DCDS is a direct and simplified description of elements that are changing in the spectral data due to the perturbation.

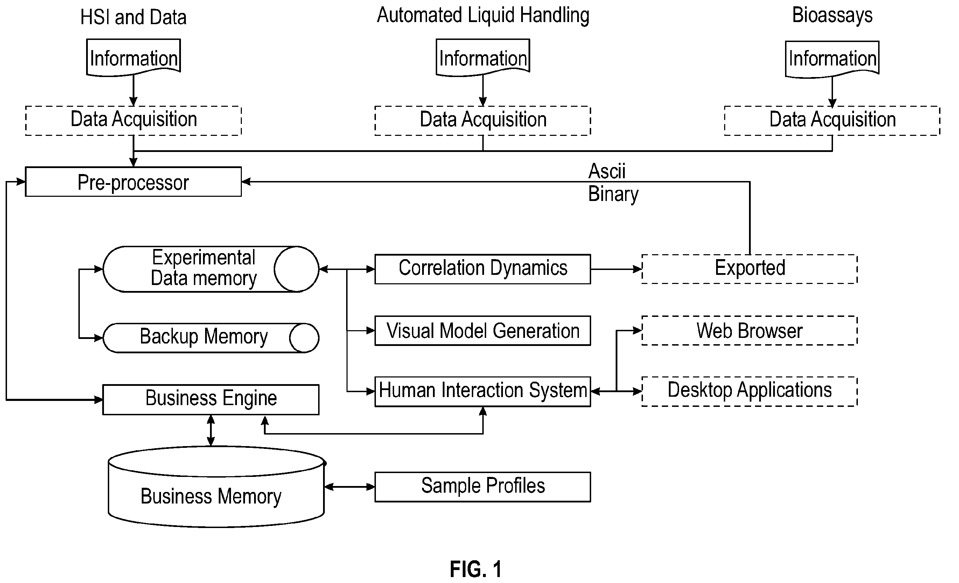

According to some embodiments, for example as shown in FIG. 1, a system for performing data analysis can include at least the components shown for performing functions of methods described herein. Data may be acquired from a plurality of sources, and may contain information related to HSI images acquired with a QCL transmission microscope, information from automated liquid handling systems, and information from bioassays. The acquired data can be provided to one or more computing units, including pre-processors and processors, for analysis. Modules can be provided to perform or manage analysis of the data. Information from the modules may also be implemented on, or exported to, web browsers, mobile applications, or desktop applications. Such modules can include a correlation dynamics module, a visual model generator module, and/or a human interaction module. The human interaction module may be provided, for example, as a web browser, mobile application, or desktop application. The modules may be in communication with one another. In some embodiments, the modules may be implemented in software (e.g., subroutines and code). For example, the modules may be stored in memory and/or data storage, such as experimental memory and/or backup memory, and executed by a processor. The processor may include a business engine, having pre-programmed rules and instructions to act on the acquired data. The business engine may communicate with a business memory, which stores sample profiles for use in the data analysis according to the methods described herein. In some aspects, some or all of the modules may be implemented in hardware (e.g., an Application Specific Integrated Circuit (ASIC), a Field Programmable Gate Array (FPGA), a Programmable Logic Device (PLD), a controller, a state machine, gated logic, discrete hardware components, or any other suitable devices), firmware, software, and/or a combination thereof. Additional features and functions of these modules according to various aspects of the subject technology are further described in the present disclosure.

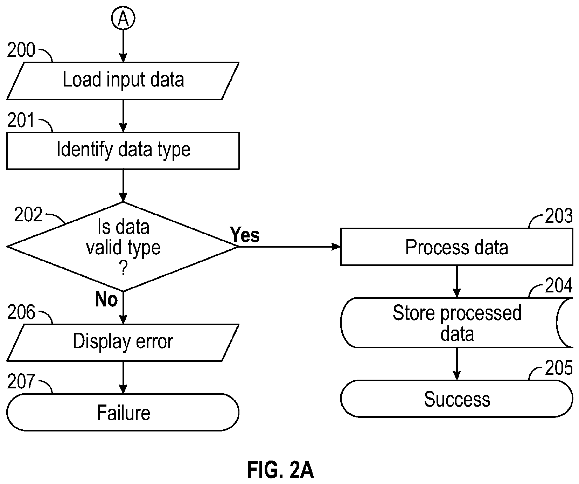

According to some embodiments, for example as shown in FIG. 2A, a method for verifying and preparing acquired data can be performed. As shown in FIG. 2A, input data loaded 200 and provided to a processor, such as the processors shown in FIG. 1. The type of data is identified 201, and a determination 202 is made as to whether the data is a valid type. If the data is a valid type, then the data is processed 203 and stored 204, and the verification and preparation of the acquired data is determined as a success 205. However, if the data is not determined to be a valid type, an error is displayed 206 and the verification and preparation is determined as a failure 207. The data can be converted and/or stored when the verification is a success, or rejected with an error displayed to a user when the verification is a failure.

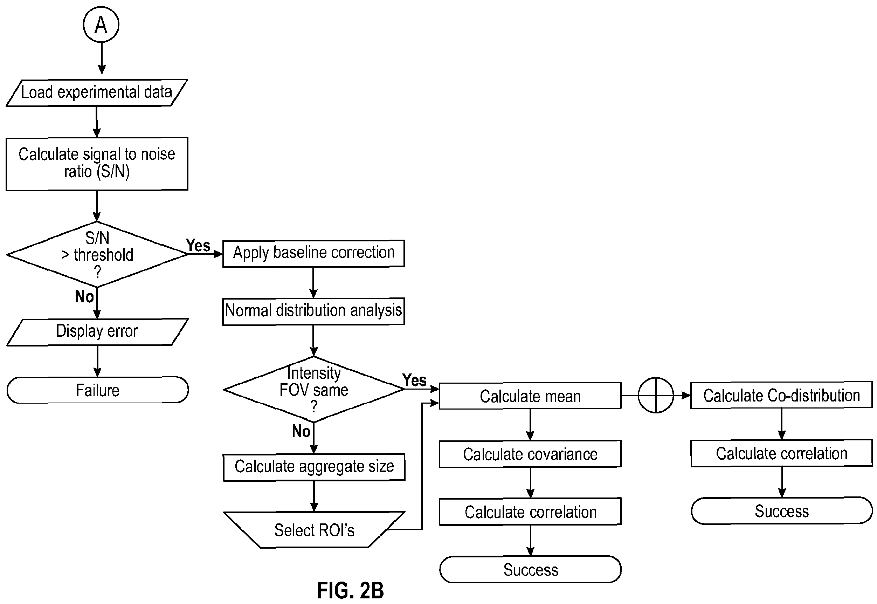

According to some embodiments, for example as shown in FIG. 2B, a method for analyzing acquired data can be performed. The type of data is verified for adequate signal-to-noise ratio relative to a threshold. Based on the verification, the data can be subject to analysis or smoothing filter process before the analysis.

According to some embodiments, for example as shown in FIG. 2B, the data can be analyzed in operations that include applying a baseline correlation, performing a normal distribution analysis, determining the intensity of the field of view, calculating aggregate size, selecting regions of interest, calculating a mean, calculating a covariance, calculating correlations, and calculating co-distributions.

Data manipulation can include auto recognition of regions of interest (ROI) for the discrimination of particulates and solution. The size and number of the particulates can be determined to ascertain population distribution of particulates. Data manipulation can be performed to ensure compliance such as S/N ratio determination, baseline correction, determine water vapor content, and determine signal intensity of the elements of interest within the spectral region studied. Data output for statistical analysis can be simplified using, inter alia, the Design of Experiment approach. The intensity and spectral position of the elements of interest can be output as comma delimited files (*.csv). Covariance, or dynamic spectral data sets can be generated based on the perturbation of the sample of interest, the output of which can be used for further analysis. For example, data output can be provided in a format that facilitates merging with other bioanalytical results for comparability assessment and sourced by: perturbation type, excipient, protein therapeutic, protein concentration, temperature, date of acquisition, and/or bioanalytical technique. This approach would allow for the statistical analysis to be performed for all of the experiments that were carried-out under similar conditions. More importantly, the results of the DOE analysis would be a standalone document ready for final reporting and allow for decision making.

According to some embodiments, methods and systems described herein can apply a correlation function to the covariance or the dynamic spectral data to generate the synchronous and asynchronous plots, as described above. The changes (e.g., peak intensities) in the spectral data that are in-phase with one another can be correlated as obtained in the synchronous plot. The elements that change in the spectral data can be determined. The overall greatest intensity change in the spectral data can be determined. The overall smallest intensity change in the spectral data can be determined. The minimum number of underlying spectral contribution in a broad band such as the amide band for proteins and peptides can be determined for curve fitting analysis, which allows for the determination of secondary structure composition. The resolution of the spectral region being studied can be enhanced, particularly for broad bands in the spectra. Moreover, by analyzing the synchronous and asynchronous plots, the order to events may be determined.

The changes (e.g., peak intensities) in the spectral data that are out-of-phase from one another can be correlated as obtained in the asynchronous plot. From the asynchronous plot, the order of events that describe in molecular detail the protein behavior may be obtained. A detailed evaluation of the plots could be performed to ascertain the order of events. Alternatively or in combination, this process can be automated. A joint variance function can be applied to the covariance or dynamic spectral data to generate the merged asynchronous plot which can be interpreted directly to determine the order of events. This method can alternatively be used to validate the above interpretations for the description of the molecular behavior of a protein which is a complex description.

Evidence of deamidation as a result of thermal stress may be obtained by analyzing out-of-phase correlation of the peaks in the asynchronous plots. When there is a correlation of peaks that exhibit out-of-phase intensity changes in the asynchronous plot, it may be determined that deamidation has occurred. A machine learning approach can be implemented as a long term solution to the complexity of the attributes needed to be correlated and solved.

EXAMPLE STUDIES

A developability and comparability assessment of the systems and methods for use in monitoring and determining deamidation in proteins was performed using assessment of three NIST mAbs (standard and candidate RM 8671 and 8670, respectively) using different concentration ranges: (low) 1.0 - 1.5 $\mu$g/$\mu$L, (intermediate) 2.0 - 2.4

μg/μL, (intermediate - high) 2.8 - 10 μg/μL. The NIST mAb (lot No. 14HB-D-002) is an IgG1κ isotype with a molecular weight of 150 KDa, a homo-dimer comprised of two heavy and two light chain subunits containing inter- and intra-chain disulfide bonds. In addition, the protein has a post-translational modification (PTM) involving an N-linked glycosylation site at $N_{300}$ located to the FC region.

The particular NIST mAbs protein samples used in the assessment are: PDS NIST mAb (RM 8671), a sample of the NIST mAb RM 8671 that was stored at the Protein Dynamic Solutions facilities in Puerto Rico when electricity and other infrastructure was destroyed during Hurricane Maria in 2017, and thus underwent extreme thermal stress; NIST mAb (RM 8671), a sample of NIST supplied mAb RM 8671 that was not exposed to thermal stress; and NIST mAb Candidate (8670), a therapeutic candidate antibody supplied by NIST.

The protein samples studied have a theoretical molar extinction coefficient (ε) at a $\lambda_{max}$ = 280 nm determined to be 212,270 $M^{-1}$ $cm^{-1}$. Dilution series of the NIST mAb samples were performed using the 12.5 mM L-Histidine buffer at pH 6.00. A concentration determination was also performed on the samples. The diluted NIST mAb samples along with the appropriate reference 12.5 mM L-Histidine buffer at pH 6.00 were used for the concentration determination by UV spectroscopy. UV spectra of the diluted NIST mAb (RM 8671 and 8670) samples were acquired using a Jasco (Tokyo, JP) model V-630 spectrophotometer and Starna (Essex, UK) demountable quartz cells model DMV-Bio with a 0.2 mm path-length at room temperature (24 °C). Two scans were co-added within the spectral region of 235 - 320 nm at a scan rate of 400 nm/min and a data pitch of 1.0 nm. A single point baseline correction was performed at 320 nm for all of the spectra collected. Origin 7 professional software from MicroCal was used to render the desired plots and analysis.

For the experimental design, predetermined amounts, such as 1 μL, of each sample with the respective reference was applied to a pre-defined well on a custom designed $CaF_2$ slide cell. The coordinates were provided for the automated image acquisition, while maintaining and thermal control of the slide cell. Care was taken to collect backgrounds at each temperature to eliminate potential coherence effects due to the Quantum Cascade Lasers.

A real-time Hyperspectral Imaging Quantum Cascade Laser Transmission Microscope (QCLTM) was used to perform automated image acquisition of the array of protein samples in solution under strict thermal control of a custom heated slide holder and slide cell. The path-length for each sample in the array was known allowing for quantitative analysis, such as the analysis described in PCT/US2017/014338. HSI raw spectral data for each sample protein was captured with the QCLTM, and the HSI data were evaluated for the presence of particles/aggregates. Further, mean spectral data was determined and baseline corrected for each protein solution sampled, and subsequent 2D IR correlation and Co-distribution plots were generated were further evaluation of deamidation events.

Upon examination of the HSI acquired for the sample proteins, none or fewer than 5 particles were observed. Differences observed were due to the extent to which deamidation impacts stability of the mAb. For example, the assessment ascertained the event of deamidation of asparagine $N_{318}$ localized to the FC domain at low concentration (1.0 - 1.5 μg/μL) due to thermal stress within the NIST mAb candidate and the PDS NIST mAb that was subject to extended high temperatures during Hurricane Maria in Puerto Rico. Also, at higher mAb concentrations the colloidal stability of the NIST mAb standard (RM 8671) and candidate (RM 8670) changes, which may also be an indication of deamidation but requires further evaluation. Finally, the NIST mAb standard was observed to have greater stability than that of the NIST mAb candidate.

Aggregates were visualized using the HSI acquired for each protein solution sampled in the array. In the data set used in the study, <5 or no aggregates were observed. Furthermore, the buffer 12.5 mM L-Histidine at pH 6.0 was also aggregate free. Based on the optical setup, any aggregates that were detected were in the 4.3 μm - 2.0 mm size range.

Three quantum cascade lasers, which provide enhanced signal to noise ratios (SNR), allowed for the use of a linear response microbolometer focal plane array (480 x 480 pixels) detector. For spatial resolution, a low magnification objective (4x) with a numerical aperture (NA) of 0.3 NA within a 2 x 2 $mm^2$ field of view (FOV) providing a pixel size of 4.25 x 4.25 μm spatial resolution was used. The QCL IR spectra were collected at 4 $cm^{-1}$ resolution within the spectral region of 1780-1450 $cm^{-1}$ for each protein sample in the array. To prevent coherence effects due to QCL fluctuations, the background was collected at each set temperature once thermal equilibrium (4 min) was achieved. Typical HSI acquisition times were 0.4 min for each sample within the array.

The raw spectral data were saved as comma delimited files (*.csv). The analysis and plots were generated from the raw data whenever needed. From the raw data, QCL IR overlays and 2D IR correlation plots were generated. The deamidation assessment module used to perform the analysis in the assessment study included a cursor feature to allow for the unbiased cross peak intensity changes and position, which is beneficial for the determination of the sequential order of molecular events.

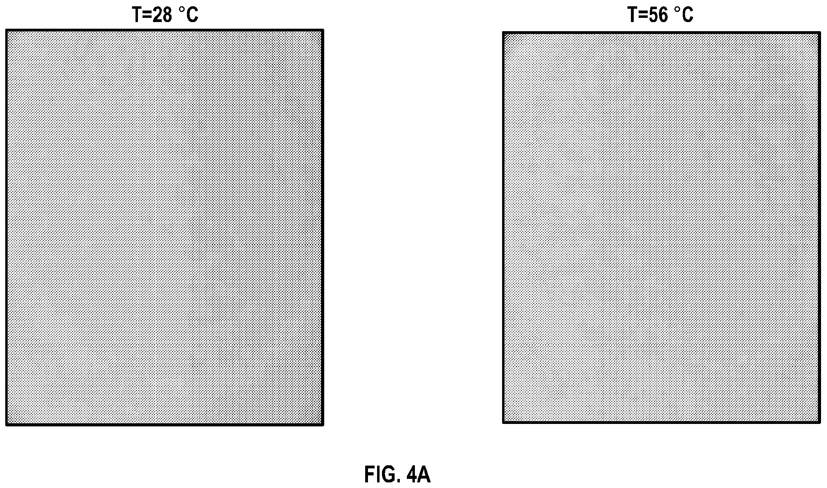

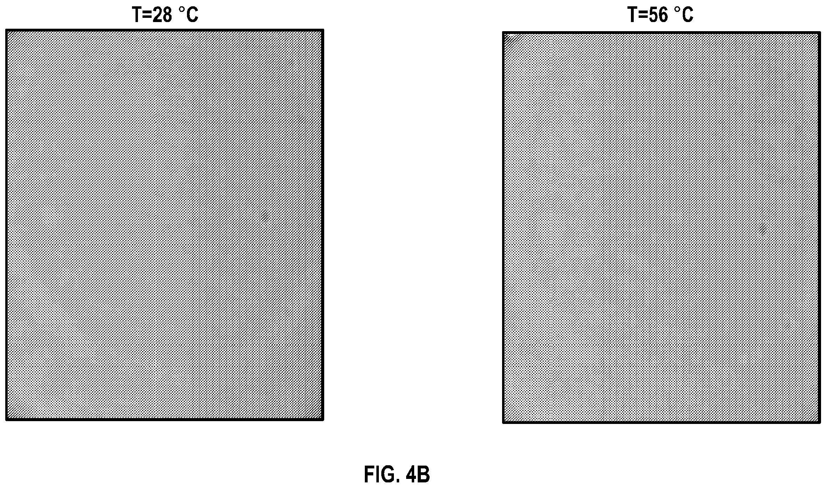

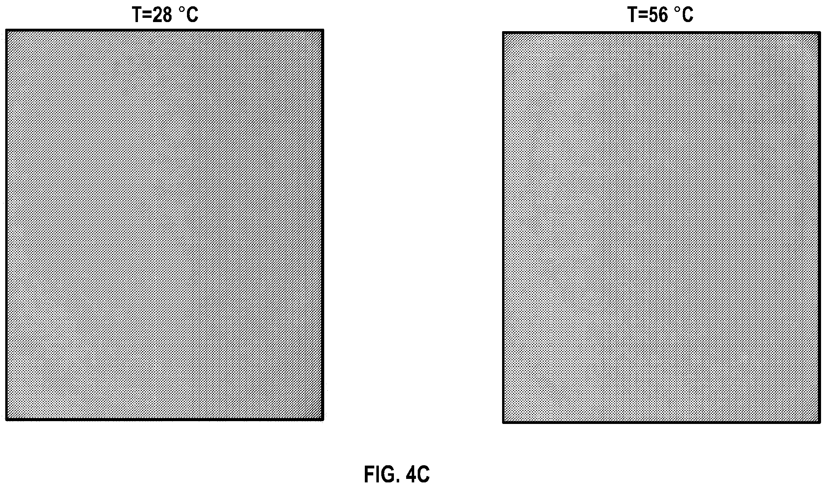

FIGS. A-4C illustrate hyperspectral images acquired within the MID IR spectral region of 1780-1450 $cm^{-1}$ and temperature range of 28-56°C with 4 °C temperature intervals for each protein sample in the array. FIG. 4A shows the hyperspectral images acquired for PDS NIST mAb RM 8671 at 1 μg/μL at 28 °C and 56 °C. F. FIG. 4B shows the hyperspectral images acquired for NIST mAb RM 8671 at 2 μg/μL at 28 °C and 56 °C. FIG. 4C shows the hyperspectral images acquired for NIST mAb Candidate RM 8670 at 2.4 μg/μL at 28 °C and 56 °C. The HSI and background acquisition were done when a set temperature was reached after a 4 min equilibration period. Each HSI was comprised of 223,000 QCL IR spectra. Each mean spectrum at its defined temperature represents a mean of 223,000 spectra within a 2 x 2 mm$^2$ FOV. The FOV matches the diameter of the well for each sample in the array.

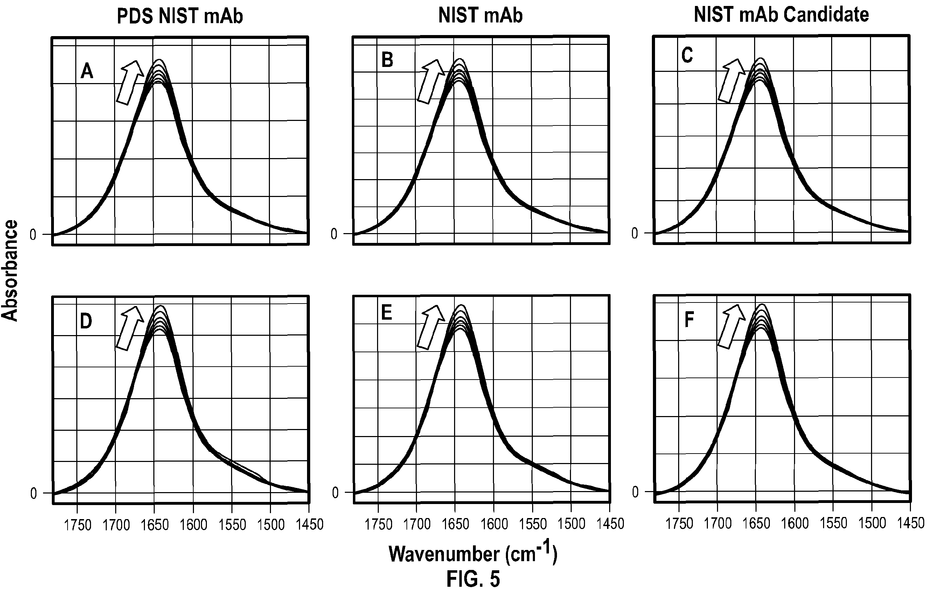

QCL IR overlays were then generated for each NIST mAb within the array, and were only baseline corrected. FIG. 5 illustrates QCL IR spectral overlays of the amide I and II bands with overlapping L-Histidine and H$_2$O absorption in the spectral region of 1780 - 1450 cm$^{-1}$ within the temperature range of 28-56 °C with 4°C temperature intervals: 28°C, 32°C, 36 °C, 40 °C, 44 °C, 48 °C, 52 °C, 56°C. The PDS NIST mAb standard (RM 8671), NIST mAb standard (RM 8671), and NIST mAb candidate (RM 8670) samples were studied at two different concentrations. The top row in FIG. 5 represents the QCL IR spectral overlays for concentrations of 1 - 1.5 μg/μL, and the bottom row shows overlays for concentrations of 2 - 2.5 μg/μL. In general, the low concentration within the range was the PDS NIST and NIST mAb standards (RM 8671) and the high concentration within the range was the NIST mAb candidate (RM 8670). Each protein sample had a low temperature mean spectrum of 28°C.

Difference spectra were then generated using the low temperature mean spectrum at 28°C for each protein sample. As a result, the changes in intensity and peak shifts within the spectral region of interest can be analyzed and therefore represent the behavior of the protein in solution due to the thermal stress.

Table 1 provides a summary of the backbone vibrational modes and positions used in the assessment:

| Table 1. Secondary structure band assignments in H$_2$O | | | |
|---|---|---|---|
| item | secondary structure | position (cm$^{-1}$) | comment |
| 1 | β-turns | 1695-1670 | observed to exhibit the lowest molar extinction coefficient |
| 2 | loop/hinges | 1667-1660 | highly flexible |
| 3 | α-helix | 1650-1657 | highest molar extinction coefficient |
| 4 | β-sheet | 1625-1638 | usually observed as a single component |
| 5 | β-sheet | 1625-1638, 1695-1685 | antiparallel when peaks are correlated with each other |
| 6 | aggegation | 1608-1624 | typically observed as a shoulder in the amide I band |

Table 2 provides a summary of the side chain modes and positions used in the assessment:

| Table 2. Assignment of amino acid side chains in H$_2$O | | | | | |
|---|---|---|---|---|---|
| item | side chain | code | vibrational mode | position (cm$^{-1}$) | comment |
| 1 | Tyr | **Y** | $\nu$(C=C) | 1518 | immediate surroundings |
| 2 | Lys | **K** | $\delta_s$ (NH$_3^+$) | 1526 | pH, H-bonding, salt bridge interactions, flexibility |
| 3 | Glu | **E** | $\nu$(COO-) | 1543-1560 | **pH, H-bonding, deamidation, salt bridge, cation binding, flexibility** |
| 4 | Asp | **D** | $\nu$(COO-) | 1570-1574 | **pH, H-bonding, deamidation, salt bridge, cation binding, flexibility** |
| 5 | His | **H** | $\nu$(C=C) | 1596-1603 | pH, H-bonding, Zn$^{2+}$ coordination |
| 6 | C-term end | | $\nu$(COO-) | 1598 | stability of the C-terminal end |
| 7 | Gin | **Q** | $\delta$ (NH$_2$) | 1586-1607 | **H-bonding, deamidation, flexibility** |
| 8 | Asn | **N** | $\delta$ (NH$_2$) | 1612-1618 | **H-bonding, deamidation, flexibility** |
| 9 | Lys | **K** | $\delta_{as}$ (NH$_3^+$) | 1625-1629 | pH, H-bonding, salt bridge interactions, flexibility |
| 10 | Arg | **R** | $\nu_s$ (CN$_3$H$_5^+$) | 1633 | pH, H-bonding, salt bridge interactions, flexibility |
| 11 | Gin | **Q** | $\nu$(C=O) | 1670 | H-bonding, flexibility |
| 12 | Arg | **R** | $\nu_{as}$ (CN$_3$H$_5^+$) | 1673 | H-bonding, salt bridge interactions, flexibility |
| 13 | Asn | **N** | $\nu$(C=O) | 1678 | H-bonding, flexibility |

(continued)

| Table 2. Assignment of amino acid side chains in $H_2O$ | | | | | |
|---|---|---|---|---|---|
| item | side chain | code | vibrational mode | position (cm$^{-1}$) | comment |
| 14 | p-Ar | **F, Y** | | 1740-1730 | hydrophobic |
| 15 | p-Ar | **F, Y** | | 1720-1715 | interaction |
| 16 | p-Ar | **F, Y** | | 1708-1700 | $\pi$- $\pi$ stacking |

The band positions used for the comparability assessment represent a mean average of all of the 2D IR correlation peaks determined for the entire data set studied for NIST mAb standard RM 8671 and NIST mAb candidate RM 8670. For the amide I band within 1700 - 1600 cm$^{-1}$, mainly due to C=O stretches, with minor contributions of C-N stretches and to a lesser extent N-H deformation modes; are sensitive to conformational changes. In general for all three mAbs, the QCL IR spectra are comprised of: the $\beta$-turns (1693.1 cm$^{-1}$), hinge loops (1665.2 cm$^{-1}$), $\alpha$-helices (1665.2 cm$^{-1}$) and $\beta$-sheets (1635.6 cm$^{-1}$). These secondary structures are commonly observed for IgGs. For the side chain modes there are some vibrational modes that overlap within the amide I band and others are located within the amide II band (1600-1500 cm$^{-1}$). The following side chain modes are located just prior to the amide I band: three p-substituted aromatic peaks that represent both phenylalanine and tyrosine side chains (1748.7, 1726.7 and 1705.0 cm$^{-1}$), glutamine $\nu$(C=O) (1670.0 cm$^{-1}$), asparagine $\nu$(C=O) (1678.3 cm$^{-1}$) and $\delta$(NH$_2$) (1612.7 cm$^{-1}$), and lysine $\delta_{as}$(NH$_3^+$) (1621.0 cm$^{-1}$). Side chain modes located within the amide II band are: glutamine $\delta$(NH$_2$) (1591.0 cm$^{-1}$), histidine $\nu$(C=C) (1600.1 cm$^{-1}$), aspartate $\nu$(COO$^-$) (1572.0 cm$^{-1}$) and two different glutamates one of which is presumably involved in salt-bridge interactions $\nu$(COO$^-$) (1540.7 and 1559.0 cm$^{-1}$), lysine $\delta_s$(NH$_3^+$) (1525.0 cm$^{-1}$) and finally the tyrosine at (1519.0 cm$^{-1}$). The arginine and tryptophan vibrational modes may also be considered.

Upon subtraction of the low temperature mean spectrum from all subsequent mean spectra the contribution of $H_2O$ and all protein vibrational modes that were not perturbed by the thermal stress were subtracted, allowing for the evaluation of only the changes that occurred in the spectral region of interest (1780 - 1450 cm$^{-1}$) upon thermal stress. The detailed molecular evaluation of the protein in solution was obtained by performing 2D IR correlation analysis.

A correlation function was applied to generate two distinct plots: (1) the synchronous plot, which provided the overall intensity changes within the spectral region of interest and (2) the asynchronous plot, which provided enhanced resolution and the sequential order of molecular events that occurred as a function of the thermal stress. Furthermore, the asynchronous plot provided detailed correlation of peaks that exhibit out-of-phase intensity changes, which were indicative of deamidation. For example, as detailed below, the assessment observed decreased intensity for asparagine at 1612.7 cm$^{-1}$ associated $\delta$(NH$_2$) vibrational mode due to deamidation, while a concomitant increase in intensity was observed for the aspartate intensity at 1572.0 cm$^{-1}$ $\nu$(COO$^-$) vibrational mode.

The overall thermal stability of the studied proteins was also assessed. Overall thermal stability was determined using thermal dependence plots by assessing the onset of the thermal transition temperature. QCL IR peak position maxima within the spectral region of 1780-1450 cm$^{-1}$ as a function of temperature in the range from 28-56 °C were observed for: i) NISST mAbs RM 8671 and RM 8670 alone at concentrations of 1-1.5 $\mu$g/$\mu$L, 2-2.4 $\mu$g/$\mu$L, and 2.8 or 10 $\mu$g/$\mu$L; ii) NIST mAbs 86781 and RM 8670 at concentrations of 1-1.5 $\mu$g/$\mu$L, 2-2.4 $\mu$g/$\mu$L, and 2.8 or 10 $\mu$g/$\mu$L, plus references; and iii) references of deionized $H_2O$ and 12.5 mM L-Histidine buffer at pH 6.0. The PDS NIST mAb standard (RM8671) at low concentration, NIST mAb standard (RM8671), NIST mAb candidate (RM 8670), at low concentrations in the range between 1-2.8 $\mu$g/$\mu$L exhibited the same onset of peak shift at 50 °C. However, the NIST Candidate (RM8670) at 10 $\mu$g/$\mu$L exhibited less stability, with the onset peak shift occurring at 32.5 °C. In the case of deionized $H_2O$, no shift in the peak maxima was observed across the temperature range, while for the 12.5 mM L-Histidine buffer the onset of the thermal transition was observed at 52.5 °C, indicating it is therefore more stable than the NIST mAbs. This suggests that the changes observed for the NIST mAbs were due to their intrinsic behavior due to the thermal stress.

Aggregation events were also monitored in the assessment, however, no aggregation during the thermal stress was observed for any of the three NIST mAb (RM8671 and 8670) samples under the conditions examined.

As described in more detail below, the assessment further monitored the spectral data to detect and analyze deamidation events. The assessment focused analysis on the cross peaks associated with the asparagine side chain carbonyl stretching mode within the amide ($\nu$C=O) at 1678.3 cm$^{-1}$ and amide bending mode ($\delta$ NH$_2$) at 1612.7 cm$^{-1}$, and the aspartate carboxylate stretching mode ($\nu$COO$^-$) at 1572.0 cm$^{-1}$. A confirmed correlation between these peaks was determined to establish deamidation occurrence for the samples (NIST mAb, RM 8671 and the NIST mAb candidate 8670).

The description of the behavior of the proteins in solution was provided by determining the sequential order of molecular events during the thermal stress (28-56°C) for each sample within the array.

The experimental approach was not to determine overall thermal transition temperature of the three NIST mAb standards (RM 8671 and 8670) protein samples, but instead to determine the differences in stability of the three proteins examined and if deamidation was observed. The thermal transition temperature can be determined using well established procedures if desired. For this study, the data was separated based on low (1.0 - 1.5 $\mu g/\mu L$) intermediate (2.0 - 2.4 $\mu g/\mu L$) and intermediate to high concentration (2.8 - 10.0 $\mu g/\mu L$) of protein to establish and understand the differences in sensitivity due to concentration for such a discrete event that can cause changes in stability in the protein and potentially reduce efficacy, as discussed above. Second, the study mapped the region where the deamidation has occurred for the protein in solution under thermal stress. Finally, the study determined stability based on the extent of deamidation and thermal stability based on the sequential order of molecular events.

EXAMPLE 1

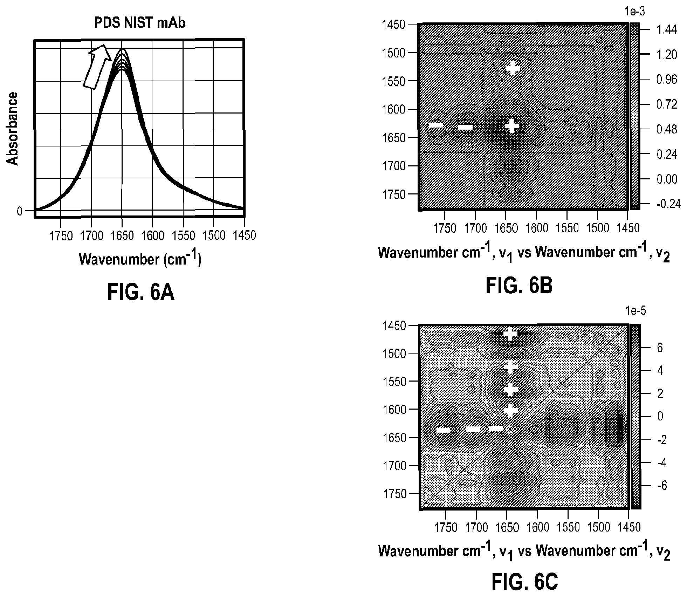

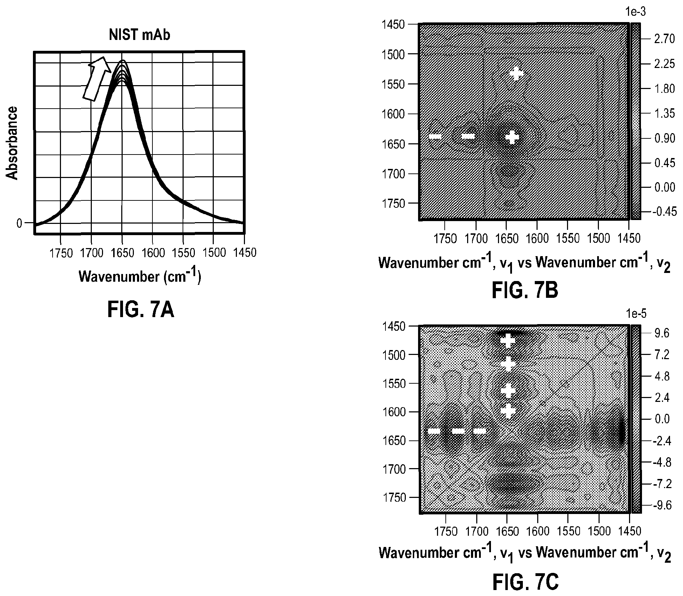

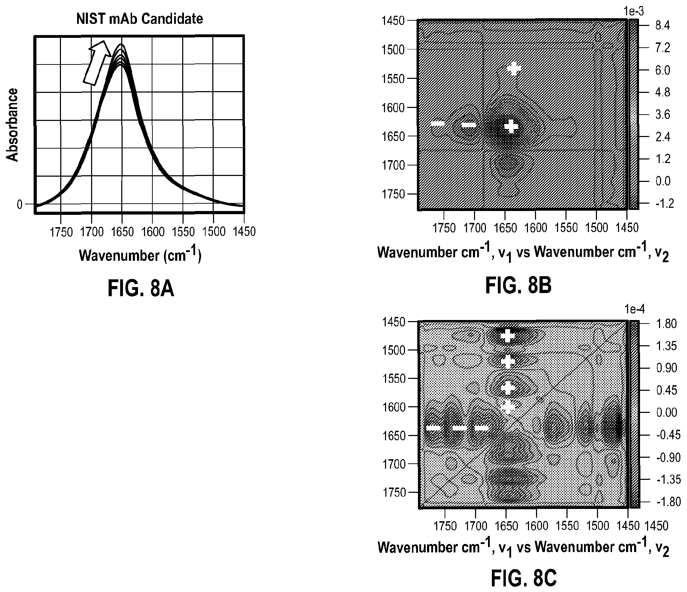

A comparative 2D IR correlation spectroscopy analysis within the spectral region of 1780-1450 cm$^{-1}$ for: PDS NIST mAb at 1 $\mu g/\mu L$, NIST mAb at 1 $\mu g/\mu L$ and NIST mAb Candidate at 1.5 $\mu g/\mu L$ in 12.5 mM L-Histidine at pH 6.0 thermally stressed within the temperature range of 28-56°C was conducted using the methods and systems described herein. QCL IR spectral overlays of amide I and amide II bands within the spectral region of 1780 - 1450 cm$^{-1}$ corresponding to the temperature range of 28-56 °C were generated for each of the three studied sample proteins. From these spectral overlays, 2D IR correlation was used to generate synchronous plots and asynchronous plots corresponding to the temperature range of 28-56 °C. FIG. 6A shows the QCL spectral overlay of amide I and amide II bands within the spectral region of 1780 - 1450 cm$^{-1}$ corresponding to the temperature range of 28-56 °C for the PDS NIST mAb sample. FIG. 6B shows the synchronous plot and FIG. 6C shows the asynchronous plot generated based on the QCL spectral overlay data shown in FIG. 6A. FIG. 7A shows the QCL spectral overlay of amide I and amide II bands within the spectral region of 1780 - 1450 cm$^{-1}$ corresponding to the temperature range of 28-56 °C for the NIST mAb sample. FIG. 7B shows the synchronous plot and FIG. 7C shows the asynchronous plot generated based on the QCL spectral overlay data shown in FIG. 7A. FIG. 8A shows the QCL spectral overlay of amide I and amide II bands within the spectral region of 1780 - 1450 cm$^{-1}$ corresponding to the temperature range of 28-56 °C for the NIST mAb Candidate sample. FIG. 8B shows the synchronous plot and FIG. 8C shows the asynchronous plot generated based on the QCL spectral overlay data shown in FIG. 8A.

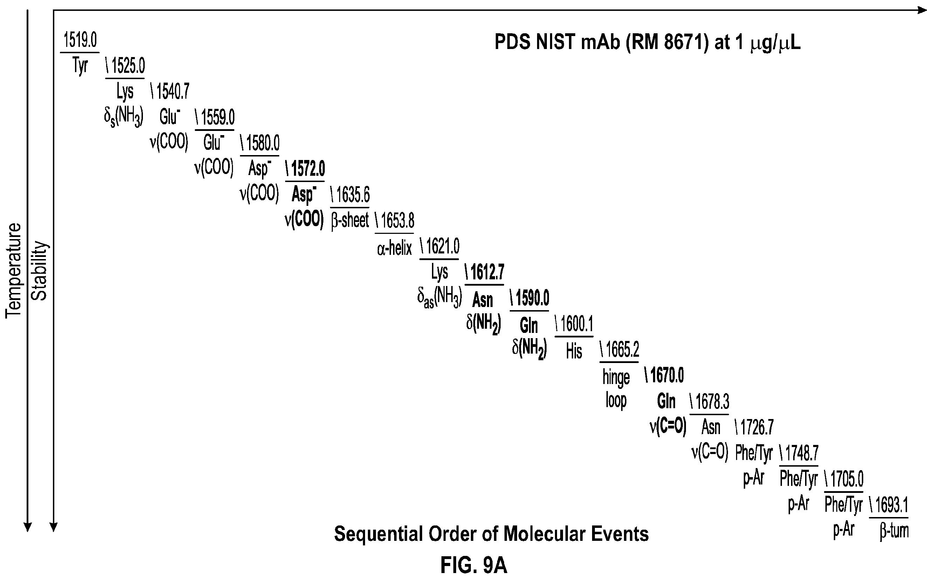

The behavior of the three mAb samples at the low concentration ranges (1.0 - 1.5 $\mu g/\mu L$) upon thermal stress was derived from an analytical interpretation of the 2D IR correlation plots. As shown in FIG. 9A, a sequential order of events for PDS NIST mAb was derived from an analysis of the synchronous and asynchronous plots shown in FIGS. 6B, 6C, using Noda's rules as described herein. As shown in FIG. 9A, the sequential order of molecular events were as follows for the PDS NIST mAb (RM 8671): the tyrosine residues (1519.0 cm$^{-1}$) followed by lysines $\delta_s(NH_3^+)$ (1525.0 cm$^{-1}$), then two types of glutamates $\nu(COO^-)$ at 1540.7 and 1559.0 cm$^{-1}$, followed by two types aspartates $\nu(COO-)$ at 1580.0 cm$^{-1}$ and 1572.0 cm$^{-1}$, presumably involved in hydrogen bonding or salt bridge interactions with the tyrosines and lysines that are located in the vicinity, $\beta$-sheets (1635.6 cm$^{-1}$) followed by the helical regions (1653.8 cm$^{-1}$) (observed for all mAbs at low concentration), then the lysines $\delta_{as}(NH_3^+)$ (1621.0 cm$^{-1}$) followed by the asparagine side chain modes $\delta(NH_2)$ (1612.7 cm$^{-1}$) and the glutamine $\delta(NH_2)$ (1591.0 cm$^{-1}$) followed by the histidine v(C=C) (1600.1 cm$^{-1}$) presumably all of these residues most be in close proximity to each other, then the hinge loops (1665 cm$^{-1}$), followed by the glutamine v(C=O) (1670.0 cm$^{-1}$) and asparagine side chain mode v(C=O) (1678.3 cm$^{-1}$) then followed by phenylalanines and tyrosine p-substituted aromatic ring modes (1726.7, 1748.7, 1705.0 cm$^{-1}$) suggesting a change in the mAbs aqueous solvent accessibility due to partial unfolding near 56°C and finally the $\beta$-turns (1693.1 cm$^{-1}$) are perturbed. These final molecular events are shared amongst all mAbs.

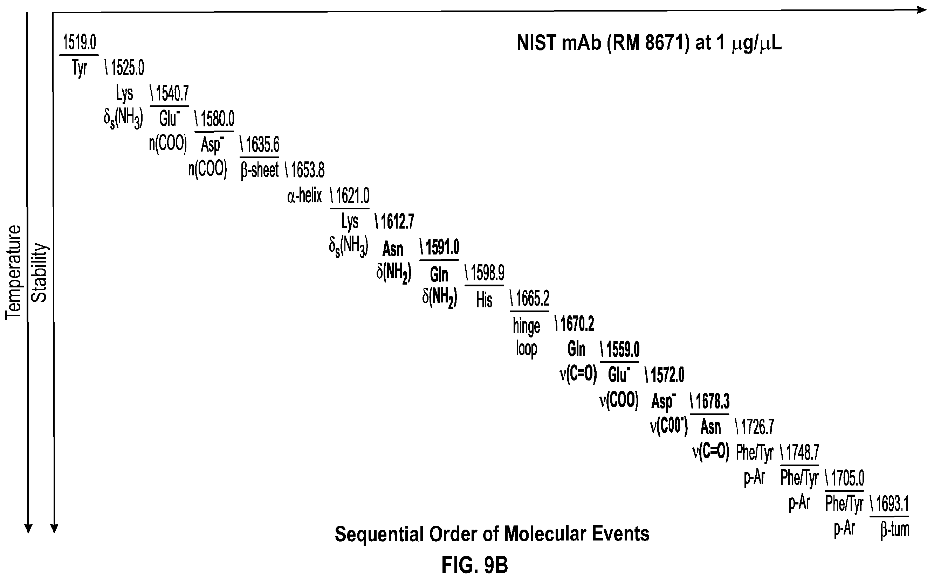

FIG. 9B shows the sequential order of events for NIST mAb, derived from the synchronous and asynchronous plots shown in FIGS. 7B, 7C. As shown in FIG. 9B, the sequential order of events for the NIST mAb (RM 8671) was as follows: the tyrosine residues (1519.0 cm$^{-1}$) followed by lysines $\delta_s(NH_3^+)$ (1525.0 cm$^{-1}$), then glutamates $\nu(COO^-)$ at 1540.7 cm$^{-1}$, followed by aspartates $\nu(COO-)$ at 1580.0 cm$^{-1}$, followed by the $\beta$-sheets (1635.6 cm$^{-1}$) and helical regions (1653.8 cm$^{-1}$), then lysines $\delta_{as}(NH_3^+)$ (1621.0 cm$^{-1}$) followed by the asparagine side chain modes $\delta(NH_2)$ (1612.7 cm$^{-1}$) and the glutamine $\delta(NH_2)$ (1591.0 cm$^{-1}$) followed by the histidine v(C=C) (1600.1 cm$^{-1}$) presumably all of these residues most be in close proximity to each other, then the hinge loops (1665 cm$^{-1}$), followed by the glutamine v(C=O) (1670.0 cm$^{-1}$), then the glutamates $\nu(COO^-)$ at 1559.0 cm$^{-1}$, and the aspartates $\nu(COO^-)$ at 1572.0 cm$^{-1}$and asparagine side chain mode v(C=O) (1678.3 cm$^{-1}$) followed by phenylalanines and tyrosine p-substituted aromatic ring modes (1726.7, 1748.7, 1705.0 cm$^{-1}$) suggesting a change in the mAbs aqueous solvent accessibility due to partial unfolding near 56°C and finally the $\beta$-turns (1693.1 cm$^{-1}$) are perturbed. For the NIST mAb that did not undergo the stress associated with Hurricane Maria had two vibrational modes stabilized the glutamates $\nu(COO^-)$ at 1559.0 cm$^{-1}$, and the aspartates $\nu(COO-)$ at 1572.0 cm$^{-1}$. These are the modes associated with deamidation.

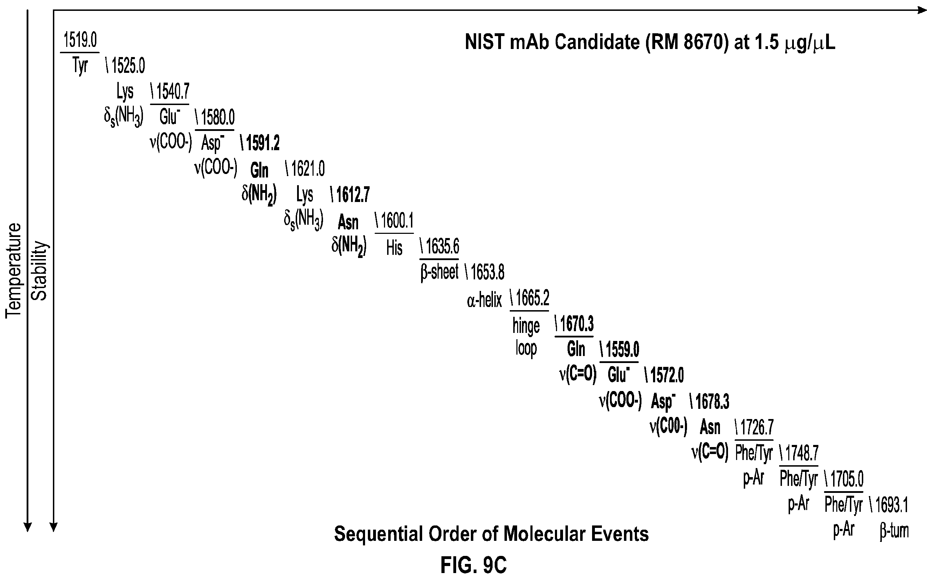

FIG. 9C shows the sequential order of events for NIST mAb candidate (RM 8670), derived from the synchronous and asynchronous plots shown in FIGS. 8B, 8C. As shown in FIG. 9C, the sequential order of events for the NIST mAb candidate (RM 8670) was as follows: the least stable are the tyrosine residues (1519.0 cm$^{-1}$) followed by lysines $\delta_s$(NH$_3$$^+$) (1525.0 cm$^{-1}$), then glutamates $\nu$(COO$^-$) at 1540.7 cm$^{-1}$, followed by aspartates $\nu$(COO-) at 1580.0 cm$^{-1}$, then the glutamine $\delta$(NH$_2$) (1591.0 cm$^{-1}$) followed by the lysines $\delta_{as}$(NH$_3$$^+$) (1621.0 cm$^{-1}$), then the asparagine side chain modes $\delta$(NH$_2$) (1612.7 cm$^{-1}$), followed by the histidine v(C=C) (1600.1 cm$^{-1}$), then the secondary structure is perturbed within the $\beta$-sheets (1635.6 cm$^{-1}$), helical regions (1653.8 cm$^{-1}$) and hinge loops (1665 cm$^{-1}$), followed by deamidation events glutamine $\nu$(C=O) (1670.0 cm$^{-1}$), then the glutamates $\nu$(COO$^-$) at 1559.0 cm$^{-1}$, and the aspartates $\nu$(COO-) at 1572.0 cm$^{-1}$and asparagine side chain mode v(C=O) (1678.3 cm$^{-1}$) followed by phenylalanines and tyrosine p-substituted aromatic ring modes (1726.7, 1748.7, 1705.0 cm$^{-1}$) suggesting a change in the mAbs aqueous solvent accessibility due to partial unfolding near 56°C and finally the $\beta$-turns (1693.1 cm$^{-1}$) are perturbed.

The differences in the sequential order of molecular events was due to the stability of these NIST mAbs prior to their thermal stress. The level of confidence is high due to the repeated events observed during both the initial and final stages of the thermal stress for all three mAbs at low concentration. The cross peaks in the asynchronous plots that seem to be destabilized at different times were further analyzed, as they would be associated with the deamidation process resulting in altered domain stability of the mAbs.

EXAMPLE 2

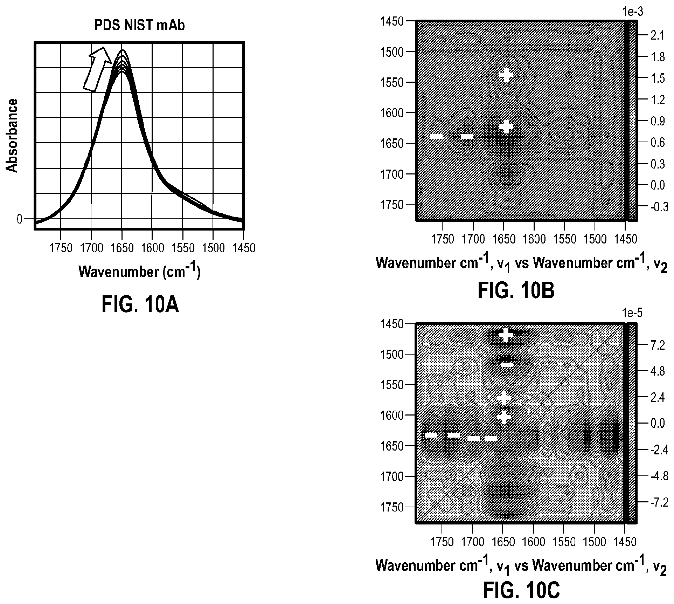

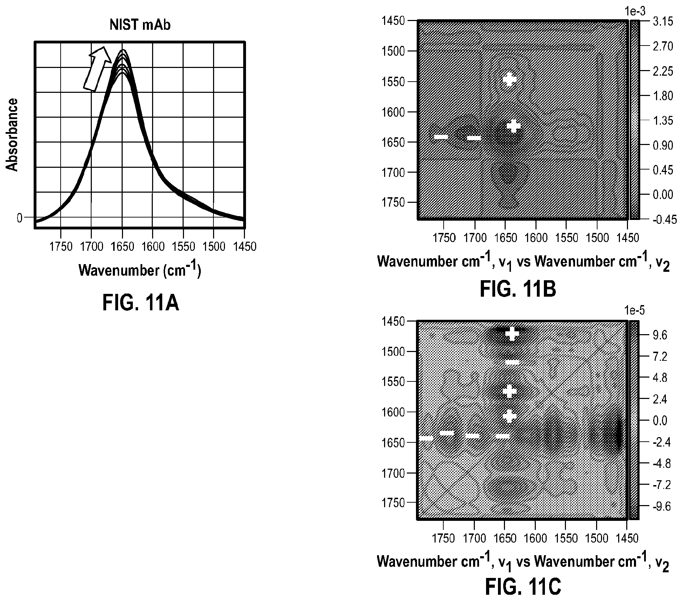

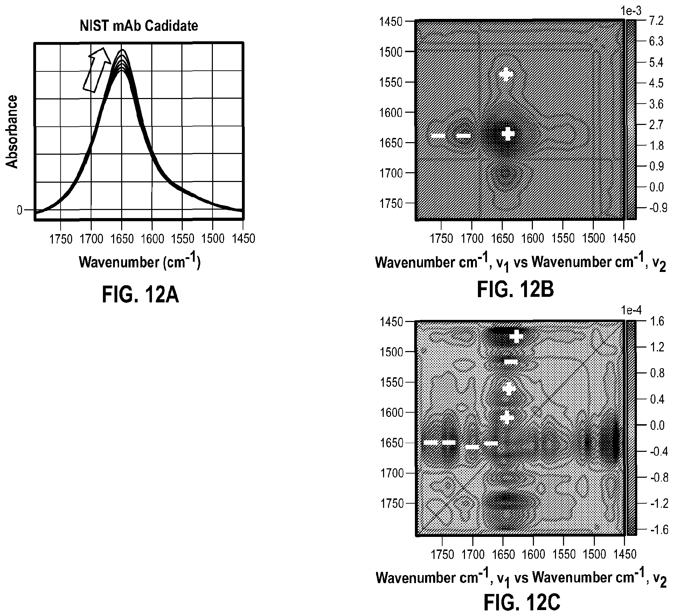

A comparative 2D IR correlation spectroscopy analysis within the spectral region of 1780-1450 cm$^{-1}$ for: PDS NIST mAb (RM 8671) at 2 $\mu$g/$\mu$L, NIST mAb (RM 8671) at 2 $\mu$g/$\mu$L and NIST mAb Candidate (RM 8670) at 2.4 $\mu$g/$\mu$L in 12.5 mM L-Histidine at pH 6.0 thermally stressed within the temperature range of 28-56°C was conducted using the methods and systems described herein. QCL IR spectral overlays of amide I and amide II bands within the spectral region of 1780 - 1450 cm$^{-1}$ corresponding to the temperature range of 28-56 °C were generated for each of the three studied sample proteins. From these spectral overlays, 2D IR correlation was used to generate synchronous plots and asynchronous plots corresponding to the temperature range of 28-56 °C. FIG. 10A shows the QCL spectral overlay of amide I and amide II bands within the spectral region of 1780 - 1450 cm$^{-1}$ corresponding to the temperature range of 28-56 °C for the PDS NIST mAb sample. FIG. 10B shows the synchronous plot and FIG. 10C shows the asynchronous plot generated based on the QCL spectral overlay data shown in FIG. 10A. FIG. 11A shows the QCL spectral overlay of amide I and amide II bands within the spectral region of 1780 - 1450 cm$^{-1}$ corresponding to the temperature range of 28-56 °C for the NIST mAb sample. FIG. 11B shows the synchronous plot and FIG. 11C shows the asynchronous plot generated based on the QCL spectral overlay data shown in FIG. 11A. FIG. 12A shows the QCL spectral overlay of amide I and amide II bands within the spectral region of 1780 - 1450 cm$^{-1}$ corresponding to the temperature range of 28-56 °C for the NIST mAb Candidate sample. FIG. 12B shows the synchronous plot and FIG. 12C shows the asynchronous plot generated based on the QCL spectral overlay data shown in FIG. 12A.

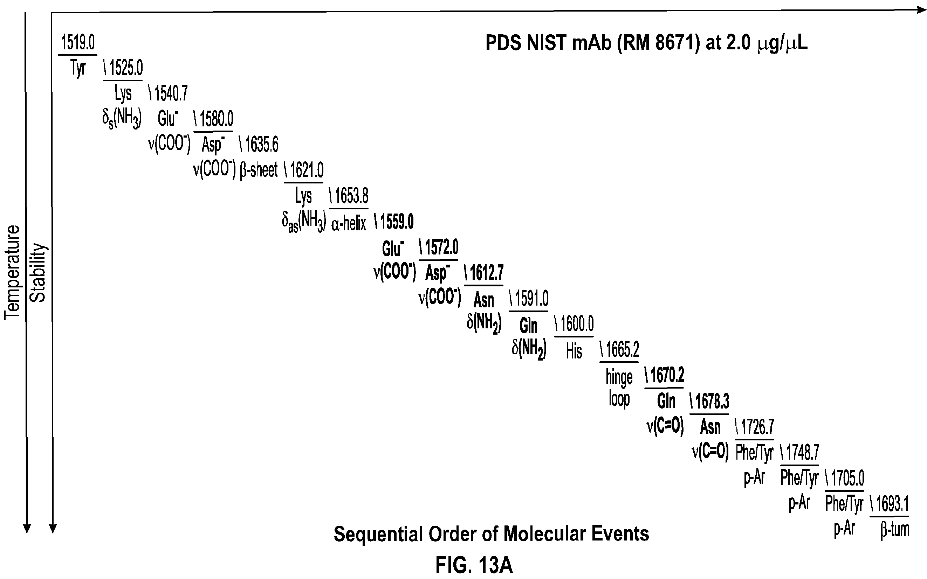

The behavior of the three mAb samples at the intermediate concentration ranges (2.0-2.4 $\mu$g/$\mu$L) upon thermal stress was derived from an analytical interpretation of the 2D IR correlation plots. As shown in FIG. 13A, a sequential order of events for PDS NIST mAb was derived from an analysis of the synchronous and asynchronous plots shown in FIGS. 10B, 10C, using Noda's rules as described herein. As shown in FIG. 13A, the sequential order of molecular events were as follows for the PDS NIST mAb (RM 8671): the tyrosine residues (1519.0 cm$^{-1}$) followed by lysines $\delta_s$(NH$_3$$^+$) (1525.0 cm$^{-1}$), then glutamates $\nu$(COO$^-$) at 1540.7 cm$^{-1}$, followed by aspartates $\nu$(COO-) at 1580.0 cm$^{-1}$, followed by the $\beta$-sheets (1635.6 cm$^{-1}$), then the lysines $\delta_{as}$(NH$_3$$^+$) (1621.0 cm$^{-1}$), followed by helical regions (1653.8 cm$^{-1}$), glutamates $\nu$(COO$^-$) at 1559.0 cm$^{-1}$, and the aspartates $\nu$(COO$^-$) at 1572.0 cm$^{-1}$, along with asparagine side chain modes $\delta$(NH$_2$) (1612.7 cm$^{-1}$) and the glutamine $\delta$(NH$_2$) (1591.0 cm$^{-1}$), suggesting the deamidation process followed by the histidine v(C=C) (1600.1 cm$^{-1}$), then the hinge loops (1665 cm$^{-1}$) are perturbed, followed by the glutamine v(C=O) (1670.0 cm$^{-1}$), then the asparagine side chain mode v(C=O) (1678.3 cm$^{-1}$) followed by phenylalanines and tyrosine p-substituted aromatic ring modes (1726.7, 1748.7, 1705.0 cm$^{-1}$) and finally the $\beta$-turns (1693.1 cm$^{-1}$) are perturbed.

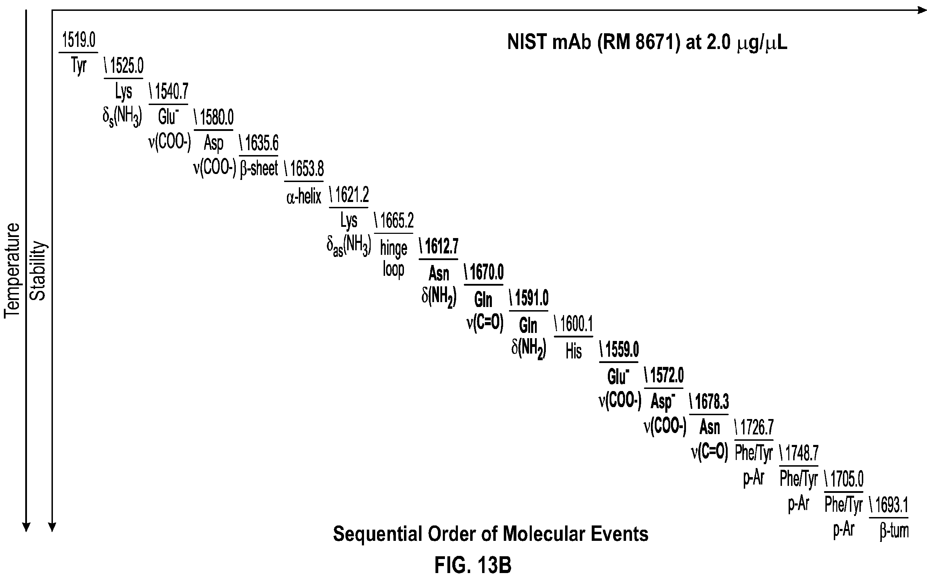

FIG. 13B shows the sequential order of events for NIST mAb, derived from the synchronous and asynchronous plots shown in FIGS. 11B, 11C. As shown in FIG. 13B, the sequential order of events for the NIST mAb (RM 8671) was as follows: the tyrosine residues (1519.0 cm$^{-1}$) followed by lysines $\delta_s$(NH$_3$$^+$) (1525.0 cm$^{-1}$), then glutamates $\nu$(COO$^-$) at 1540.7 cm$^{-1}$, followed by aspartates $\nu$(COO-) at 1580.0 cm$^{-1}$, followed by the $\beta$-sheets (1635.6 cm$^{-1}$) followed by the helical regions (1653.8 cm$^{-1}$), then the lysines $\delta_{as}$(NH$_3$$^+$) (1621.0 cm$^{-1}$), followed by the hinge loops (1665 cm$^{-1}$), then the asparagine side chain mode $\delta$(NH$_2$) (1612.7 cm$^{-1}$), followed by glutamine v(C=O) (1670.0 cm$^{-1}$), the glutamine $\delta$(NH$_2$) (1591.0 cm$^{-1}$), then the histidine v(C=C) (1600.1 cm$^{-1}$), then the glutamates $\nu$(COO$^-$) at 1559.0 cm$^{-1}$, and the aspartates $\nu$(COO$^-$) at 1572.0 cm$^{-1}$, followed by the asparagine side chain mode v(C=O) (1678.3 cm$^{-1}$) followed by phenylalanines and tyrosine p-substituted aromatic ring modes (1726.7, 1748.7, 1705.0 cm$^{-1}$) and finally the $\beta$-turns (1693.1 cm$^{-1}$) are perturbed.

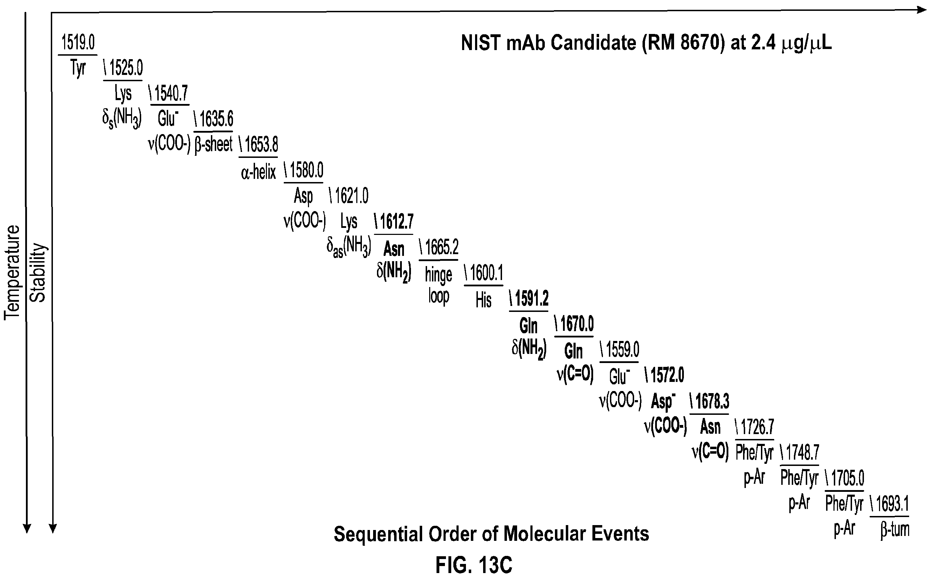

FIG. 13C shows the sequential order of events for NIST mAb candidate (RM 8670), derived from the synchronous and asynchronous plots shown in FIGS. 12B, 12C. As shown in FIG. 13C, the sequential order of events for the NIST mAb candidate (RM 8670) was as follows: the least stable are the tyrosine residues (1519.0 cm$^{-1}$), followed by lysines $\delta_s$(NH$_3$$^+$)

(1525.0 cm$^{-1}$), then glutamates $\nu$(COO$^-$) at 1540.7 cm$^{-1}$, followed by the $\beta$-sheets (1635.6 cm$^{-1}$) then the helical regions (1653.8 cm$^{-1}$), followed by the aspartates $\nu$(COO-) at 1580.0 cm$^{-1}$, the lysines $\delta_{as}$(NH$_3$$^+$) (1621.0 cm$^{-1}$), then the asparagine side chain mode $\delta$(NH$_2$) (1612.7 cm$^{-1}$), followed by the hinge loops (1665 cm$^{-1}$), then the histidine $\nu$(C=C) (1600.1 cm$^{-1}$), then glutamine $\delta$(NH$_2$) (1591.0 cm$^{-1}$), glutamine $\nu$(C=O) (1670.0 cm$^{-1}$) followed by the glutamate $\nu$(COO$^-$) at 1559.0 cm$^{-1}$, suggesting the deamidation; followed by the aspartate $\nu$(COO$^-$) at 1572.0 cm$^{-1}$, then the asparagine side chain mode $\nu$(C=O) (1678.3 cm$^{-1}$) followed by phenylalanines and tyrosine p-substituted aromatic ring modes (1726.7, 1748.7, 1705.0 cm$^{-1}$) and finally the $\beta$-turns (1693.1 cm$^{-1}$) are perturbed.

EXAMPLE 3

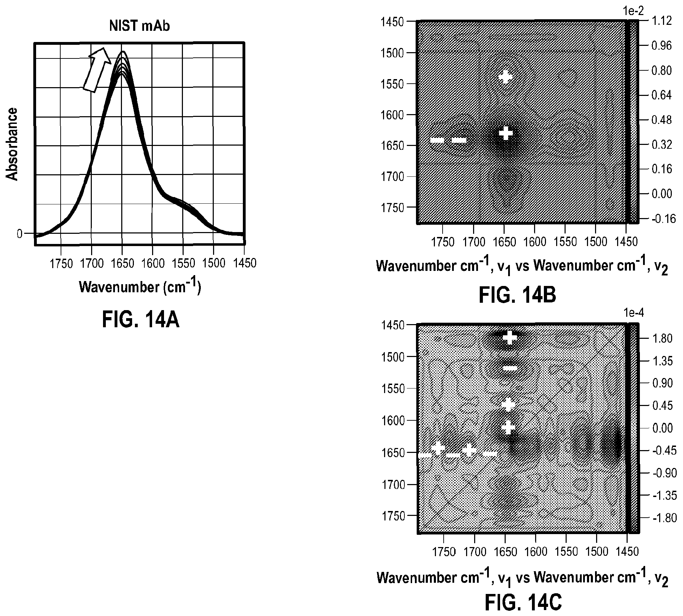

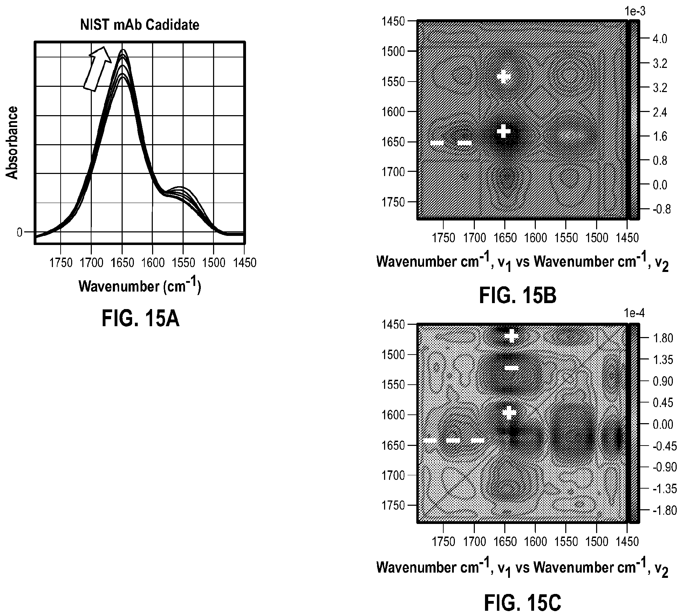

A comparative 2D IR correlation spectroscopy analysis within the spectral region of 1780-1450 cm$^{-1}$ for: NIST mAb (RM 8671) at 2.8 $\mu$g/$\mu$L and NIST mAb Candidate (RM 8670) at 10.0 $\mu$g/$\mu$L in 12.5 mM L-Histidine at pH 6.0 thermally stressed within the temperature range of 28-56°C was conducted using the methods and systems described herein. QCL IR spectral overlays of amide I and amide II bands within the spectral region of 1780 - 1450 cm$^{-1}$ corresponding to the temperature range of 28-56 °C were generated for NIST mAb (RM 8671) at 2.8 $\mu$g/$\mu$L and NIST mAb Candidate (RM 8670) at 10.0 $\mu$g/$\mu$L. From these spectral overlays, 2D IR correlation was used to generate synchronous plots and asynchronous plots corresponding to the temperature range of 28-56 °C. FIG. 14A shows the QCL spectral overlay of amide I and amide II bands within the spectral region of 1780 - 1450 cm$^{-1}$ corresponding to the temperature range of 28-56 °C for the NIST mAb sample. FIG. 14B shows the synchronous plot and FIG. 14C shows the asynchronous plot generated based on the QCL spectral overlay data shown in FIG. 14A. FIG. 15A shows the QCL spectral overlay of amide I and amide II bands within the spectral region of 1780 - 1450 cm$^{-1}$ corresponding to the temperature range of 28-56 °C for the NIST mAb Candidate sample. FIG. 15B shows the synchronous plot and FIG. 15C shows the asynchronous plot generated based on the QCL spectral overlay data shown in FIG. 15A. The higher concentration shown in the synchronous and asynchronous plot may reflect one or more of the following: (1) a change in colloidal stability of the protein due to increased concentration during thermal stress, (2) an indication of intermolecular interactions that under low protein concentration are less frequent and/or (3) that the glutamine deamidation event decreases the stability of the NIST mAb when coupled to the deamidation event of the asparagine residues that occur more readily (kinetically favored compared to glutamine).

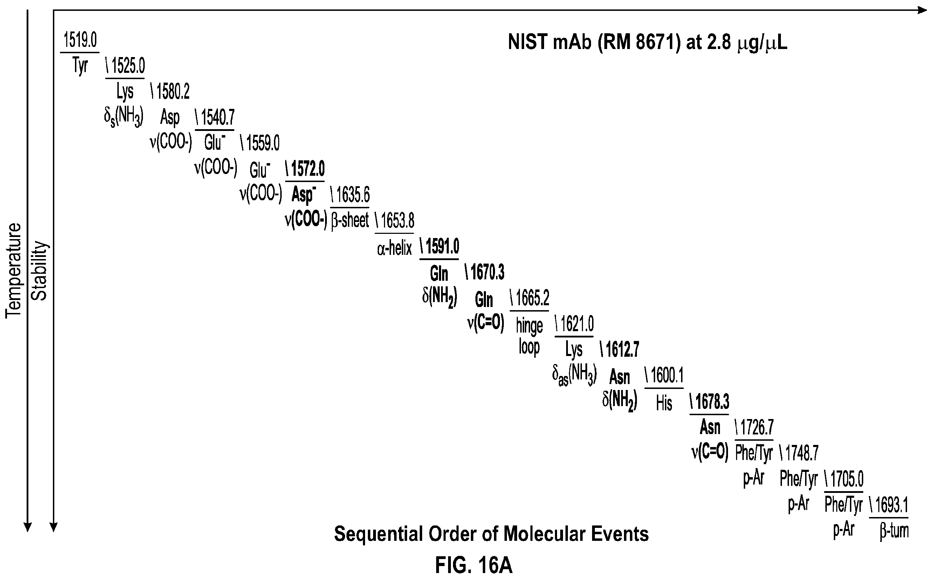

The sequential order of molecular events at intermediate and high concentrations of 2.8 and 10.0 $\mu$g/$\mu$L for NIST mAb standard (RM 8671) and NIST mAb candidate (RM 8670), respectively upon thermal stress was derived from an analytical interpretation of the 2D IR correlation plots. As shown in FIG. 16A, a sequential order of events for PDS NIST mAb was derived from an analysis of the synchronous and asynchronous plots shown in FIGS. 14B, 14C, using Noda's rules as described herein. As shown in FIG. 16A, the sequential order of events for the NIST mAb (RM 8671) at intermediate (2.8 $\mu$g/$\mu$L) was as follows: the tyrosine residues (1519 cm$^{-1}$) were perturbed first, followed by the lysines (1525 cm$^{-1}$), then the aspartates $\nu$(COO-) at 1580.0 cm$^{-1}$, two types of glutamates $\nu$(COO$^-$) at 1540.7 and 1559.0 cm$^{-1}$ and aspartates $\nu$(COO-) at 1572.0 cm$^{-1}$, presumably involved in hydrogen bonding and salt bridge interactions; then the $\beta$-sheets (1635.6 cm$^{-1}$) then the helical regions (1653.8 cm$^{-1}$) are perturbed, followed by the glutamine $\delta$(NH$_2$) (1591.0 cm$^{-1}$) and glutamine $\nu$(C=O) (1670.0 cm$^{-1}$), then the hinge loops (1665 cm$^{-1}$) are perturbed, followed by the lysines $\delta_{as}$(NH$_3$$^+$) (1621.0 cm$^{-1}$), then by asparagine side chain mode $\delta$(NH$_2$) (1612.7 cm$^{-1}$), followed by histidine $\nu$(C=C) (1600.1 cm$^{-1}$), then the asparagine side chain mode $\nu$(C=O) (1678.3 cm$^{-1}$) followed by phenylalanines and tyrosine p-substituted aromatic ring modes (1726.7, 1748.7, 1705.0 cm$^{-1}$) and finally the $\beta$-turns (1693.1 cm$^{-1}$) are perturbed.

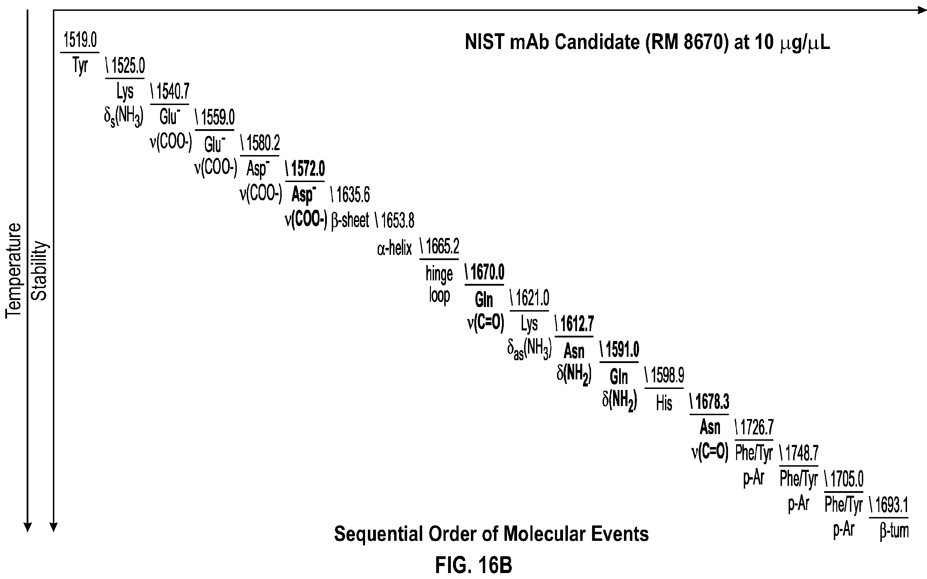

FIG. 16B shows the sequential order of events for NIST mAb candidate (RM 8670), derived from the synchronous and asynchronous plots shown in FIGS. 15B, 15C. As shown in FIG. 13C, the sequential order of events for the NIST mAb candidate (RM 8670) at high (10 $\mu$g/$\mu$L) concentration was as follows: The tyrosine residues (1519.0 cm$^{-1}$) followed by lysines $\delta_s$(NH$_3$$^+$) (1525.0 cm$^{-1}$), then two types of glutamates $\nu$(COO$^-$) at 1540.7 and 1559.0 cm$^{-1}$, followed by two types aspartates $\nu$(COO-) at 1580.0 cm$^{-1}$ and 1572.0 cm$^{-1}$, presumably involved in hydrogen bonding or salt bridge interactions with the tyrosines and lysines that are located in the vicinity, then the secondary structures are perturbed with the $\beta$-sheets (1635.6 cm$^{-1}$) followed by the helical regions (1653.8 cm$^{-1}$) and the hinge loops (1665 cm$^{-1}$), followed by the glutamine $\nu$(C=O) (1670.0 cm$^{-1}$), then the lysines $\delta_{as}$(NH$_3$$^+$) (1621.0 cm$^{-1}$), followed by the asparagine side chain mode $\delta$(NH$_2$) (1612.7 cm$^{-1}$), and glutamine $\delta$(NH$_2$) (1591.0 cm$^{-1}$); suggesting these are the stable residues that do not undergo deamidation, followed by histidine $\nu$(C=C) (1600.1 cm$^{-1}$), then the asparagine side chain mode $\nu$(C=O) (1678.3 cm$^{-1}$) followed by phenylalanines and tyrosine p-substituted aromatic ring modes (1726.7, 1748.7, 1705.0 cm$^{-1}$) and finally the $\beta$-turns (1693.1 cm$^{-1}$) are perturbed.

DETERMINATION OF DEAMIDATION INDUCED BY THERMAL STRESS

Evaluation of the asynchronous plots for the PDS NIST mAb standard (RM 8671), NIST mAb standard (RM 8671), and NIST mAb candidate (RM 8670) shown in FIGS. 6C, 7C, and 8C demonstrated the multivariate relationship that is in accordance with deamidation in proteins. Thus, the data evaluated in the assessment demonstrate that HSI using a QCL microscope and the methods disclosed herein is selective and sensitive to the determination of asparagine and glutamine deamidation induced by thermal stress.

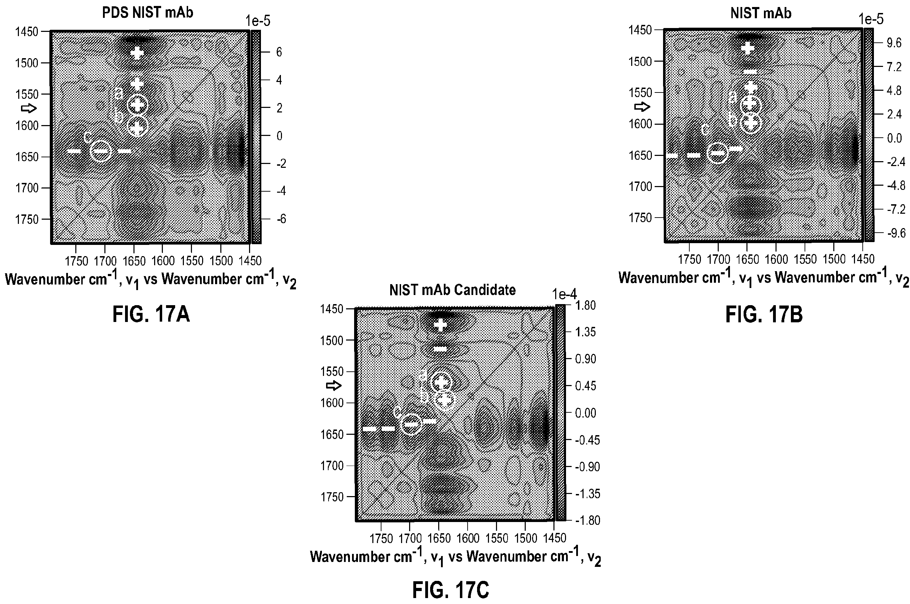

FIGS. 17A, 17B and 17C are asynchronous plots for PDS NIST mAb standard (RM 8671), NIST mAb standard (RM 8671), and NIST mAb candidate (RM 8670) corresponding to the asynchronous plots shown in FIGS. 6A, 6B, and 6C, but with additional markings to indicate evidence of observed deamidation. As shown in FIGS. 17A, 17B and 17C, deamidation at low concentration range (1-1.5 $\mu g/\mu L$) as function of thermal stress is evident by the out-of-phase correlation highlighted with white circles in the asynchronous plots. The white circles in each of FIGS. 17A, 17B and 17C designated as (a), and also indicated by the left arrows, represent the aspartate $\nu$(COO-) at 1572.0 cm$^{-1}$ intensity increase,. The white circles designated as (b) represent asparagine $\delta$(NH$_2$) at 1612.7 cm$^{-1}$. Finally, the white circles designated as (c), and also indicated by top arrow, represent the asparagine $\nu$(C=O) carbonyl stretch of the amide side chain mode at 1678 cm$^{-1}$. For both the PDS NIST mAb standard (RM 8671) and NIST mAb candidate (RM 8670), the asparagine cross peaks are observed to be less evident in the asynchronous contour plots in which deamidation has been a significant event due to the stressor condition when compared to the NIST mAb standard (RM8671).

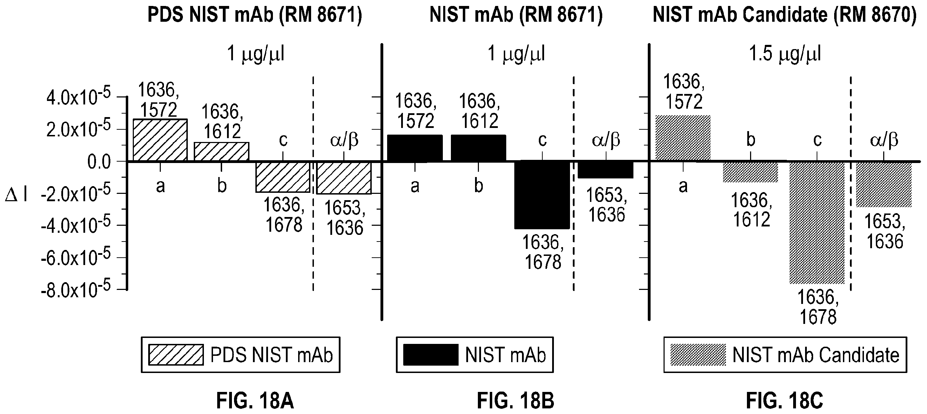

Intensity changes were determined for key cross peaks in FIGS. 17A, 17B, and 17C, and these intensity changes were used in the deamidation analyis. FIGS. 18A, 18B and 18C provide bar graphs of the intensity changes at the positions designated as (a), (b), and (c) in FIGS. 17A, 17B, and 17C, respectively. These bar graphs further show the relative stability of the beta-sheet/helical secondary structure ($\alpha/\beta$). In particular, the cross peaks located in the $\beta$-sheet that are associated with a deamidation event were monitored, with: (a) being the peak reflecting the formation of aspartate through $\nu$(COO-), (b) being the peak reflecting the loss of asparagine through $\delta$(NH$_2$) and (c) being the peak representing the perturbation of asparagine side chain $\nu$(C=O), during the conversion to aspartate in the deamidation process.

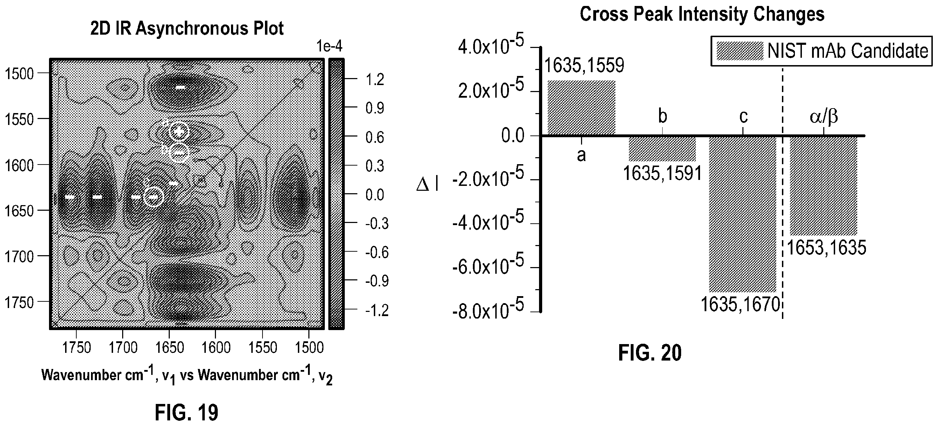

Evidence glutamine deamidation for NIST mAb Candidate at low concentration during thermal stress was also found in the assessment, indciating that evaluation of glutamine residues can also be used to identify and map deamidation events. FIG. 19 shows an asynchronous plot within the spectral region of 1780 - 1485 cm$^{-1}$ for NIST mAb Candidate at low concentration during thermal stress. Key cross peaks within the plot were monitored, and three key peaks located in the in the $\beta$-sheet that are associated with deamidation were identified: (a) the peak representing the formation of glutamate through $\nu$(COO-)), (b) the peak representing the loss of glutamine through $\delta$(NH$_2$) and (c) the peak representing perturbation of glutamine side chain $\nu$(C=O), during the conversion to glutamate.

FIG. 20 is a bar graph summarizing the ratio of intensity changes for key cross peaks identified in FIG. 19. Analyzing the intensity changes for the key cross peaks confirms the deamidation event. The results confirm the deamidation of glutamine within the NIST mAb candidate (RM 8670). This process contributes significantly to the destabilization of the mAb.

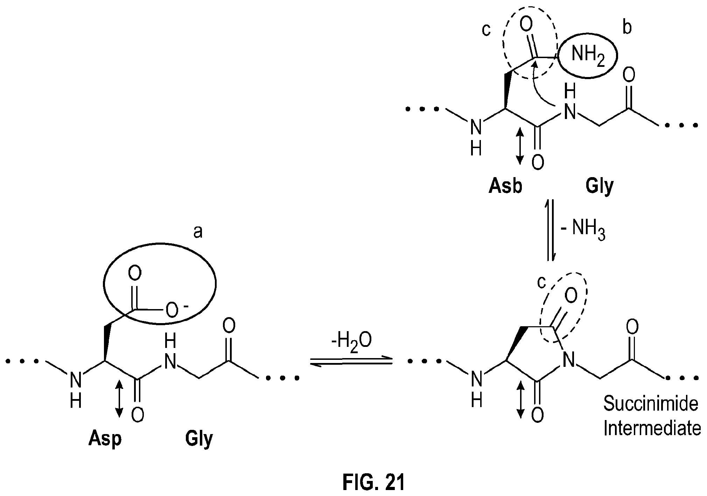

FIG. 21 is a schematic representation of the mechanism of deamidation for asparagine along with key vibrational modes that are used to monitor the event during thermal stress. QCL IR provides highly selective and sensitive detection of molecular events during deamidation. These vibrational modes become the internal probes for this process in the intact protein while in solution during stress. The intensity changes associated with a deamidation event (asparagine/glutamine) and the relative stability of the secondary structure are also monitored. Backbone $\nu$(C=O) that are affected by deamidation event include: (a) the formation of aspartate through $\nu$(COO-), (b) the loss of asparagine through $\delta$(NH$_2$) and (c) perturbation of asparagine side chain $\nu$(C=O), during the generation of the succinimide intermediate and conversion to aspartate.

Deamidation is considered as a post-translational modification that can affect the stability, structure and efficacy of a therapeutic protein and may cause aggregation which can lead to an unwanted immune response. The residues that exhibit deamidation are asparagine and to a lesser extent glutamine. Asparagine post-translational modification occurs readily when its neighboring residue (position N+1) is glycine, lowering steric hindrance for the succinimide intermediate to form, to produce aspartate or isoaspartate. The event of deamidation occurs in the absence of any enzyme and is accelerated at high pH and/or temperature. Deamidation may signal degradation of the protein within the cell, thus decreasing the therapeutics protein half-life within the cell thus potentially affecting PK/PD.

Using the systems and methods described herein allows for assessment of whether deamidation as a post-translational modification is prevalent in a protein solution, and if it affects the protein stability in solution. To do so, it is beneficial to focus the analysis on sites most likely to undergo deamidation. The examination assessment of the primary sequence of NIST mAb standard IgG1$\kappa$ described herein revealed that there are only two asparagine residues within the entire sequence of the protein that satisfy the N+1 criteria mentioned above: 1) N$_{369}$G$_{370}$ located within a $\beta$-turn that is also exposed to the aqueous environment and 2) N$_{318}$G$_{319}$ located within a 3$_{10}$ helix downstream from the N$_{300}$ glycosylated site. To discern if deamidation is present, the likelihood of identifying the Critical Quality Attribute (CQA) in the protein is in its most readily accessible site identified above. There may be other asparagine residues that may undergo deamidation such as N+1 in which the neighboring residue is alanine (A), and these may be used as well.

Further, the number of glutamates neighboring the N$_{369}$ may be destabilized by the increase in negative charge by the deamidation event. This would lead to destabilization of the $\beta$-sheet or hinge loop where the N$_{369}$ is localized, i.e. the FC domain. Another candidate is the N$_{318}$ located to the 3$_{10}$ helix within the FC domain also has a neighboring glutamate, aspartate, histidine and tyrosine residues which would account for the level of perturbation observed at low concentration.

Deamidation of glutamine residues that have the least level of steric hindrance are distributed in both the heavy chain and light chain. More importantly, they are located within the variable FAB region and even a CDR within the light chain. In contrast, the asparagine residues that may undergo deamidation readily are limited within the FC domain. Examination of the NIST mAb standard (RM 8671) glutamine residues that have a neighboring glycine residue (position Q+1) to identify the surrounding neighboring residues enabled mapping and subsequent identification of the QG responsible for the deamidation event.

IMMUNOGENICITY RISK

Bioassays have long been used to address the potential for a therapeutic protein to be immunogenic. Therapeutic proteins represent the second largest biopharmaceutical product category after vaccines. To date, the biopharma industry has addressed the potential for therapeutic proteins to induce immunogenicity or anti-drug antibody (ADA) response with the use of binding antibody type screenings collectively termed bioassays. Unfortunately, on occasions these bioassays have resulted in generating false positive or negative results. This has been the motivation for the drafting of an immunogenicity guidance by the FDA during 2016. In general, regulatory agencies worldwide have requested the implementation of an orthogonal analytical tools to validate bioassays to assess immunogenicity and or ADA risk.

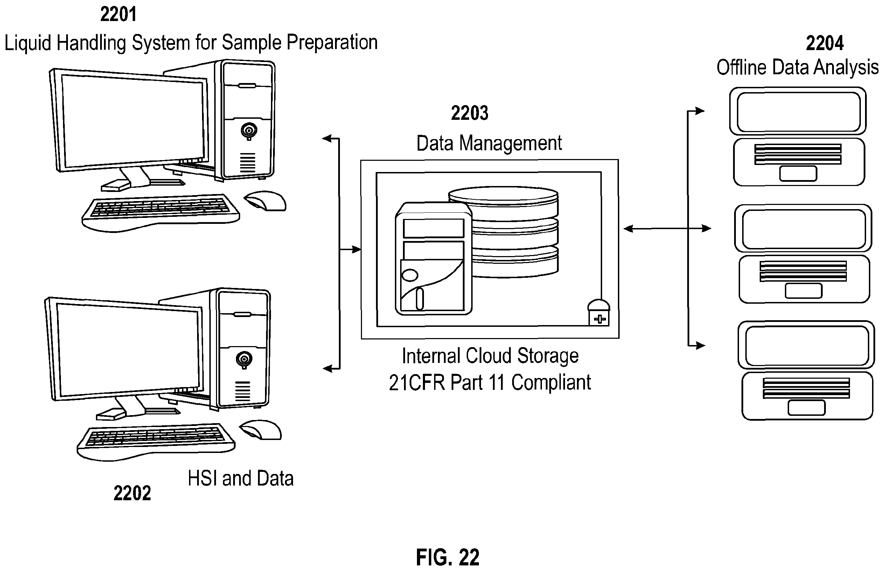

Protein aggregation is a common factor in both immunogenicity and ADA *in situ* response. Aggregation is directly measured without the use of probes and based on first principle data obtained from the platform technology used to implement the systems and methods described herein. The platform technology is comprised of a dedicated liquid handling system, a real-time Quantum Cascade Laser microscope with modified stage providing enhanced signal-to-noise ratio (SNR), slide cells, heated slide cell holder, PLC controller and computer systems implementing software modules to analyze the data and store and communicate results. As shown in FIG. 22, the platform technology may include a liquid handling system 2201 for sample preparation and a spectral imaging acquisition system 2202, such as an HSI imaging system using QCL microscopes for monitoring of an array of proteins in solution during stress. A data management system, such as a cloud storage system, may be provided that is in communication with the liquid handling system 2201 and special imaging acquisition system 2202. The data management system 2203 may also be in communication with remote computing systems 2204, allowing for remote or offline analysis of the data.

The platform technology may be implemented in an array based method to allow for the reproducible determination of aggregation induced by the therapeutic protein in human sera under physiological temperature range (37-41°C). The array based method requires minimal sample, and the results can be determined prior to first in human clinical trials, with predictive profiles for adverse events based on gender, pre-existing conditions or current medical prescriptions. This provides a predictive tool for the subsequent design of clinical trials. Furthermore, the quality and statistical robustness of the results obtained, is amenable to big data analytics and machine learning.

Orthogonal implementation of the platform technology implementing the array based method can provide a validation for the current bioassays being conducted by biopharma involving therapeutic proteins. A well designed ADA assay should be based on the rationale for the immunogenicity testing paradigm within the investigational new drug (IND) application filing stage. ADA assays are required when positive immunogenicity results are obtained.

The platform technology further provides for a validating analytical approach to existing immunogenicity assays. The process of validation involves the assessment of sensitivity, specificity, selectivity and precision requirements. The assessment of aggregation is the crux of this process, which can be ascertained by a highly selective and sensitive techniques that are statistically robust. The use of animal models for immunogenicity screening has been questioned for its transferability/applicability to humans based on animal model outcome and that of clinical trials.

The methods proposed for immunogenicity and ADA risk assessment do not present risk to patients or donors of mounting an immune response to a therapeutic protein product because the analysis is done on the sample sera, and not *in vivo*. Only 100 μL of sera are required per triplicate assay. The analysis is designed to contain the appropriate negative and positive controls.

Table 3 provides a summary of a typical assay setup in triplicates for immunogenicity comparability at different dosing levels:

TABLE 3

| [DP] | Negative control | formulation | Biosimilar | | | Innovator | | | Positive control | |
|---|---|---|---|---|---|---|---|---|---|---|
| | | | low | middle | high | low | middle | high | low | high |
| row 1 | NC1 | Formulation 1 | Biosimilar 1 | Biosimilar 1 | Biosimilar 1 | Innovator 1 | Innovator 1 | Innovator 1 | PC1 | PC1 |
| row 2 | NC1 | Formulation 1 | Biosimilar 1 | Biosimilar 1 | Biosimilar 1 | Innovator 1 | Innovator 1 | Innovator 1 | PC1 | PC1 |
| row 3 | NC1 | Formulation 1 | Biosimilar 1 | Biosimilar 1 | Biosimilar 1 | Innovator 1 | Innovator 1 | Innovator 1 | PC1 | PC1 |

Note: Triplicates generated by the same analyst.

Real-time assessments entail analyses of the samples as soon as possible after sampling, before banking of the samples. An aliquot of the human sera sample would serve as a negative control, an additional control sample would include the formulation, while a series of sera samples are exposed to varying amounts of the therapeutic protein product, as per the Immunogenicity FDA draft guidance. The analysis can also be performed in a time-defined manner to assess the presence of aggregation within the sample sera. The platform technology provides a highly selective and sensitive approach towards the direct determination of aggregates allowing for comparability assessment between biosimilar and reference material (originator). If aggregation is observed, then the extent of aggregation can be determined and followed with a titering ADA assay.

The platform technology for titering ADA assays has been designed to measure the magnitude of the ADA response by assessing the extent of aggregation in each sera sample. Aggregation events would be considered as presenting the potential of a safety risk for the patient. The event of aggregation in sera or PBMC, if persistent during the ADA titer, may also correlate to decreased efficacy. ADA assay precision will be evaluated with 3 independent preparations of the same sample per slide cell with a coefficient of variance less than 10%. The evaluation will involve ranges of low, middle and high for validation of the assay.

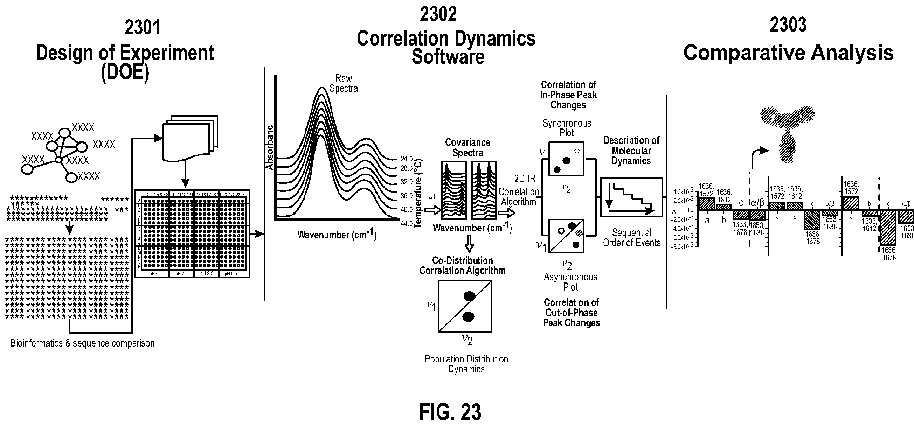

FIG. 23 is a flow chart indicating operations of an exemplary design of experiments method according to some aspects of the subject technology. As shown in FIG. 23, design of experiments techniques are applied to obtained bioinformatics and sequence comparison information. The resulting data is then subjected to spectral analysis at 2302, such as by using correlation analysis techniques as described with respect to FIG. 3. Then, the results of the spectral analysis can be subjected to comparative analysis 2303 as described herein.

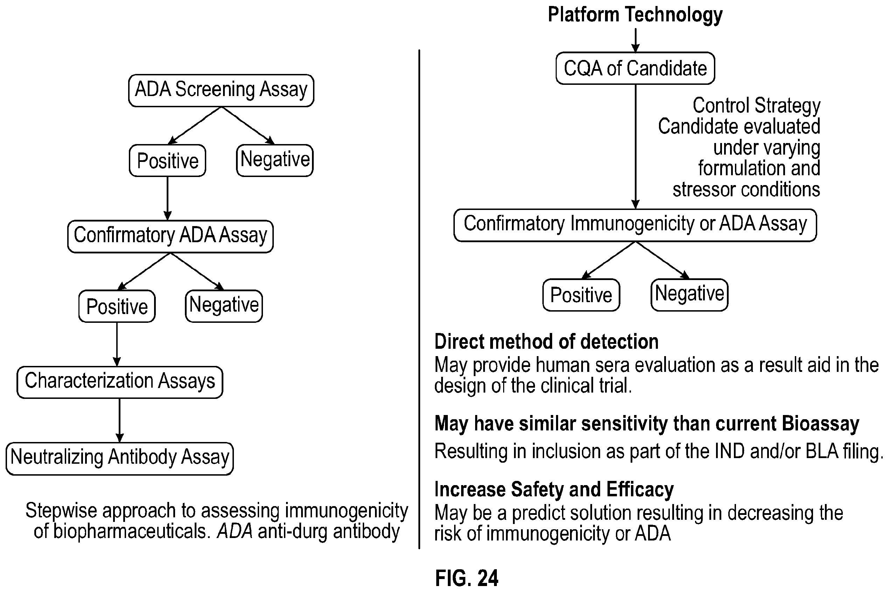

FIG. 24 is a flow chart indicating operations of exemplary methods for ADA screening and immunogenicity risk assessment.

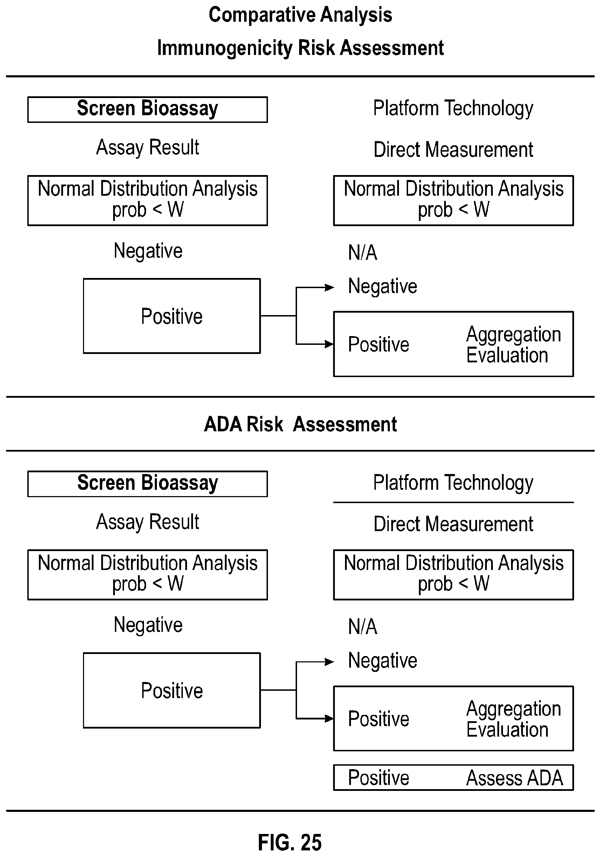

FIG. 25 is a flow chart indicating operations of an exemplary comparative analysis that may be performed using the platform technology and methods described herein.

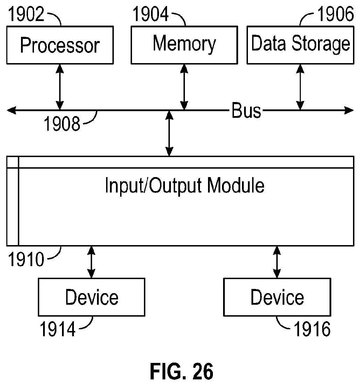

FIG. 26 is a block diagram illustrating an exemplary computer system with which a computing device (e.g., of FIG. 4) can be implemented. In certain embodiments, the computer system 1900 may be implemented using hardware or a combination of software and hardware, either in a dedicated server, or integrated into another entity, or distributed across multiple entities.

The computer system 1900 includes a bus 1908 or other communication mechanism for communicating information, and a processor 1902 coupled with the bus 1908 for processing information. By way of example, the computer system 1900 may be implemented with one or more processors 1902. The processor 1902 may be a general-purpose microprocessor, a microcontroller, a Digital Signal Processor (DSP), an Application Specific Integrated Circuit (ASIC), a Field Programmable Gate Array (FPGA), a Programmable Logic Device (PLD), a controller, a state machine, gated logic, discrete hardware components, and/or any other suitable entity that can perform calculations or other manipulations of information.

The computer system 1900 can include, in addition to hardware, code that creates an execution environment for the computer program in question, e.g., code that constitutes processor firmware, a protocol stack, a database management system, an operating system, or a combination of one or more of them stored in an included memory 1904, such as a Random Access Memory (RAM), a flash memory, a Read Only Memory (ROM), a Programmable Read-Only Memory (PROM), an Erasable PROM (EPROM), registers, a hard disk, a removable disk, a CD-ROM, a DVD, and/or any other suitable storage device, coupled to the bus 1908 for storing information and instructions to be executed by the processor 1902. The processor 1902 and the memory 1904 can be supplemented by, or incorporated in, special purpose logic circuitry.

The instructions may be stored in the memory 1904 and implemented in one or more computer program products, i.e., one or more modules of computer program instructions encoded on a computer readable medium for execution by, or to control the operation of, the computer system 1900, and according to any method well known to those of skill in the art, including, but not limited to, computer languages such as data-oriented languages (e.g., SQL, dBase), system languages (e.g., C, Objective-C, C++, Assembly), architectural languages (e.g., Java, .NET), and/or application languages (e.g., PHP, Ruby, Perl, Python). Instructions may also be implemented in computer languages such as array languages, aspect-oriented languages, assembly languages, authoring languages, command line interface languages, compiled languages, concurrent languages, curly-bracket languages, dataflow languages, data-structured languages, declarative languages, esoteric languages, extension languages, fourth-generation languages, functional languages, interactive mode languages, interpreted languages, iterative languages, list-based languages, little languages, logic-based languages, machine languages, macro languages, metaprogramming languages, multiparadigm languages, numerical analysis, non-English-based languages, object-oriented class-based languages, object-oriented prototype-based languages, off-side rule languages, procedural languages, reflective languages, rule-based languages, scripting languages, stack-based languages, synchronous languages, syntax handling languages, visual languages, wirth languages, and/or xml-based languages. The memory 1904 may also be used for storing temporary variable or other intermediate information during execution of instructions to be executed by the processor 1902.

A computer program as discussed herein does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, subprograms, or portions of code). A computer program can be deployed to be executed on one computer or on multiple computers that are located at one site or distributed across multiple sites and interconnected by a communication network. The processes and logic flows described in this specification can be performed by one or more programmable processors executing one or more computer programs to perform functions by operating on input data and generating output.

The computer system 1900 further includes a data storage device 1906 such as a magnetic disk or optical disk, coupled to the bus 1908 for storing information and instructions. The computer system 1900 may be coupled via an input/output module 1910 to various devices (e.g., devices 1914 and 1916). The input/output module 1910 can be any input/output module. Exemplary input/output modules 1910 include data ports (e.g., USB ports), audio ports, and/or video ports. In some embodiments, the input/output module 1910 includes a communications module. Exemplary communications modules include networking interface cards, such as Ethernet cards, modems, and routers. In certain aspects, the input/output module 1910 is configured to connect to a plurality of devices, such as an input device 1914 and/or an output device 1916. Exemplary input devices 1914 include a keyboard and/or a pointing device (e.g., a mouse or a trackball) by which a user can provide input to the computer system 1900. Other kinds of input devices 1914 can be used to provide for interaction with a user as well, such as a tactile input device, visual input device, audio input device, and/or brain-computer interface device. For example, feedback provided to the user can be any form of sensory feedback (e.g., visual feedback, auditory feedback, and/or tactile feedback), and input from the user can be received in any form, including acoustic, speech, tactile, and/or brain wave input. Exemplary output devices 1916 include display devices, such as a cathode ray tube (CRT) or liquid crystal display (LCD) monitor, for displaying information to the user.

According to certain embodiments, a client device and/or a server can be implemented using the computer system 1900 in response to the processor 1902 executing one or more sequences of one or more instructions contained in the memory 1904. Such instructions may be read into the memory 1904 from another machine-readable medium, such as the data storage device 1906. Execution of the sequences of instructions contained in the memory 1904 causes the processor 1902 to perform the process steps described herein. One or more processors in a multi-processing arrangement may also be employed to execute the sequences of instructions contained in the memory 1904. In some embodiments, hard-wired circuitry may be used in place of or in combination with software instructions to implement various aspects of the present disclosure. Thus, aspects of the present disclosure are not limited to any specific combination of hardware circuitry and software.

Various aspects of the subject matter described in this specification can be implemented in a computing system that includes a back end component (e.g., a data server), or that includes a middleware component (e.g., an application server), or that includes a front end component (e.g., a client computer having a graphical user interface and/or a Web browser through which a user can interact with an implementation of the subject matter described in this specification), or any combination of one or more such back end, middleware, or front end components. The components of the system 1900 can be interconnected by any form or medium of digital data communication (e.g., a communication network). Examples of communication networks include a local area network and a wide area network.

The term "machine-readable storage medium" or "computer readable medium" as used herein refers to any medium or media that participates in providing instructions to the processor 1902 for execution. Such a medium may take many forms, including, but not limited to, non-volatile media, volatile media, and transmission media. Non-volatile media include, for example, optical or magnetic disks, such as the data storage device 1906. Volatile media include dynamic memory, such as the memory 1904. Transmission media include coaxial cables, copper wire, and fiber optics, including the wires that comprise the bus 1908. Common forms of machine-readable media include, for example, floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM, a FLASH EPROM, any other memory chip or cartridge, or any other medium from which a computer can read. The machine-readable storage medium can be a machine-readable storage device, a machine-readable storage substrate, a memory device, a composition of matter effecting a machine-readable propagated signal, or a combination of one or more of them.

As used herein, a "processor" can include one or more processors, and a "module" can include one or more modules.

In an aspect of the subject technology, a machine-readable medium is a computer-readable medium encoded or stored with instructions and is a computing element, which defines structural and functional relationships between the instructions and the rest of the system, which permit the instructions' functionality to be realized. Instructions may be executable, for example, by a system or by a processor of the system. Instructions can be, for example, a computer program including code. A machine-readable medium may comprise one or more media.

As used herein, the word "module" refers to logic embodied in hardware or firmware, or to a collection of software instructions, possibly having entry and exit points, written in a programming language, such as, for example C++. A software module may be compiled and linked into an executable program, installed in a dynamic link library, or may be written in an interpretive language such as BASIC. It will be appreciated that software modules may be callable from other modules or from themselves, and/or may be invoked in response to detected events or interrupts. Software instructions may be embedded in firmware, such as an EPROM or EEPROM. It will be further appreciated that hardware modules may be comprised of connected logic units, such as gates and flip-flops, and/or may be comprised of programmable units, such as programmable gate arrays or processors. The modules described herein are preferably implemented as software modules, but may be represented in hardware or firmware.

It is contemplated that the modules may be integrated into a fewer number of modules. One module may also be separated into multiple modules. The described modules may be implemented as hardware, software, firmware or any combination thereof. Additionally, the described modules may reside at different locations connected through a wired or wireless network, or the Internet.

In general, it will be appreciated that the processors can include, by way of example, computers, program logic, or other substrate configurations representing data and instructions, which operate as described herein. In other embodiments, the processors can include controller circuitry, processor circuitry, processors, general purpose single-chip or multi-chip microprocessors, digital signal processors, embedded microprocessors, microcontrollers and the like.

Furthermore, it will be appreciated that in one embodiment, the program logic may advantageously be implemented as one or more components. The components may advantageously be configured to execute on one or more processors. The components include, but are not limited to, software or hardware components, modules such as software modules, object-oriented software components, class components and task components, processes methods, functions, attributes, procedures, subroutines, segments of program code, drivers, firmware, microcode, circuitry, data, databases, data structures, tables, arrays, and variables.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Furthermore, to the extent that the term "include," "have," or the like is used in the description or the claims, such term is intended to be inclusive in a manner similar to the term "comprise" as "comprise" is interpreted when employed as a transitional word in a claim.

The word "exemplary" is used herein to mean "serving as an example, instance, or illustration." Any embodiment described herein as "exemplary" is not necessarily to be construed as preferred or advantageous over other embodiments.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. All structural and functional equivalents to the elements of the various configurations described throughout this disclosure that are known or later come to be known to those of ordinary skill in the art are intended to be encompassed by the subject technology. Moreover, nothing disclosed herein is intended to be dedicated to the public regardless of whether such disclosure is explicitly recited in the above description.

**Claims**

1. A computer-implemented method for processing data representing a characteristic of proteins, peptides, and/or peptoids, the method comprising:

    obtaining spectral data, taken using a quantum cascade laser microscope, of the proteins, peptides, and/or peptoids without the use of probes or additives with respect to an applied perturbation;
    applying two-dimensional correlation analysis to generate an asynchronous correlation plot for the proteins, peptides, and/or peptoids; and
    identifying in the asynchronous correlation plot at least one peak associated with deamidation of the proteins, peptides, and/or peptoids.

2. The method of claim 1, further comprising using the at least one peak to determine an order of a distributed presence of spectral intensity changes with respect to the applied perturbation.

3. The method of claim 2, wherein using the at least one peak comprises:
determining, for two wavenumbers $v_1$ and $v_2$, whether the at least one peak corresponding to the two wavenumbers has a positive value.

4. The method of claim 2, wherein using the at least one peak comprises:
determining, for two wavenumbers $v_1$ and $v_2$, whether the at least one peak corresponding to the two wavenumbers has a negative value.

5. The method of claim 1, further comprising identifying a plurality of peaks in the asynchronous correlation plot, and determining a deamidation event has occurred when there is a correlation of peaks that exhibit out-of-phase intensity changes.

6. The method of claim 1, wherein obtaining the spectral data includes analyzing side chain modes of the proteins, peptides, and/or peptoids as internal probes.

7. The method of claim 1, further comprising performing a two-dimensional codistribution analysis on the spectral data.

8. The method of claim 1, further comprising:
applying two-dimensional correlation analysis to generate a synchronous correlation plot for the proteins, peptides, and/or peptoids.

9. The method of claim 8, further comprising determining a sequential order of molecular events from the asynchronous correlation plot and synchronous correlation plot.

10. The method of claim 9, further comprising determining the extent of deamidation based on the sequential order of molecular events.

11. The method of claim 8, further comprising determining the stability of domains in the proteins, peptides, and/or peptoids.

12. A system for processing data representing a characteristic of proteins, peptides, and/or peptoids, the system comprising:

    a data acquisition module configured to obtain spectral data, taken using a quantum cascade laser microscope, of the proteins, peptides, and/or peptoids without the use of probes or additives with respect to an applied perturbation; and
    a correlation analysis module configured to:

        apply two-dimensional correlation analysis to generate an asynchronous correlation plot for the proteins, peptides, and/or peptoids; and
        identify in the asynchronous correlation plot at least one peak associated with deamidation of the proteins, peptides, and/or peptoids.

13. The system of claim 12, wherein the correlation analysis module is configured to:
use the at least one peak to determine an order of a distributed presence of spectral intensity changes with respect to the applied perturbation.

14. The system of claim 13, wherein using the at least one peak comprises:
determining, for two wavenumbers $v_1$ and $v_2$, whether the at least one peak corresponding to the two wavenumbers has a positive value.

15. The system of claim 13, wherein using the at least one peak comprises:
determining, for two wavenumbers $v_1$ and $v_2$, whether the at least one peak corresponding to the two wavenumbers has a negative value.

16. The system of claim 12, wherein obtaining the spectral data includes analyzing side chain modes of the proteins, peptides, and/or peptoids as internal probes.

17. The system of claim 12, wherein the correlation analysis module is configured to:
apply two-dimensional correlation analysis to generate a synchronous correlation plot for the proteins, peptides, and/or peptoids.

18. The system of claim 17, wherein the correlation analysis module is further configured to:

determine a sequential order of molecular events from the asynchronous correlation plot and synchronous correlation plot; and
determine the extent of deamidation based on the sequential order of molecular events.

19. Non-transitory computer-readable medium comprising instructions which, when executed by one or more computers, cause the one or more computers to:

obtain spectral data, taken using a quantum cascade laser microscope, of the proteins, peptides, and/or peptoids without the use of probes or additives with respect to an applied perturbation;
apply two-dimensional correlation analysis to generate an asynchronous correlation plot for the proteins, peptides, and/or peptoids; and
identify in the asynchronous correlation plot at least one peak associated with deamidation of the proteins, peptides, and/or peptoids.

**Patentansprüche**

1. Computerimplementiertes Verfahren zum Verarbeiten von Daten, die eine Eigenschaft eines Proteins, Peptids und/oder Peptoids darstellen, wobei das Verfahren umfasst:

Erfassen von Spektraldaten, aufgenommenen unter Verwendung eines Quantenkaskadenlaser-Mikroskops, der Proteine, Peptide und/oder Peptoide ohne Verwendung von Sonden oder Additiven in Bezug auf eine angewendete Störung;
Anwenden einer zweidimensionalen Korrelationsanalyse, um ein asynchrones Korrelationsdiagramm für die Proteine, Peptide und/oder Peptoide zu erzeugen; und
Identifizieren in dem asynchronen Korrelationsdiagramm mindestens eines mit Desamidierung der Proteine, Peptide und/oder Peptoide assoziierten Peaks.

2. Verfahren nach Anspruch 1, ferner umfassend Benutzen des mindestens einen Peaks, um eine Reihenfolge einer verteilten Präsenz von spektralen Intensitätsänderungen in Bezug auf die angewandte Störung zu bestimmen.

3. Verfahren nach Anspruch 2, wobei das Benutzen des mindestens einen Peaks umfasst:
Bestimmen, für zwei Wellenzahlen $v_1$ und $v_2$, ob der mindestens eine Peak, der den zwei Wellenzahlen entspricht, einen positiven Wert hat.

4. Verfahren nach Anspruch 2, wobei das Benutzen des mindestens einen Peaks umfasst:
Bestimmen, für zwei Wellenzahlen $v_1$ und $v_2$, ob der mindestens eine Peak, der den zwei Wellenzahlen entspricht, einen negativen Wert hat.

5. Verfahren nach Anspruch 1, ferner umfassend Identifizieren einer Vielzahl von Peaks in dem asynchronen Korrelationsdiagramm und Bestimmen, dass ein Desamidierungsereignis aufgetreten ist, wenn es eine Korrelation von Peaks gibt, die außerphasige Intensitätsänderungen zeigen.

6. Verfahren nach Anspruch 1, wobei das Erfassen der Spektraldaten, analysieren der Seitenkettenmoden der Proteine, Peptide und/oder Peptoide als interne Sonden einschließt.

7. Verfahren nach Anspruch 1, ferner umfassend Durchführen einer zweidimensionalen Co-Verteilungs-Analyse an den spektralen Daten.

8. Verfahren nach Anspruch 1, ferner umfassend:
Anwenden einer zweidimensionalen Korrelationsanalyse, um ein synchrones Korrelationsdiagramm für die Proteine, Peptide und/oder Peptoide zu erzeugen.

9. Verfahren nach Anspruch 8, ferner umfassend Bestimmen einer sequenziellen Abfolge molekularer Ereignisse aus dem asynchronen Korrelationsdiagramm und dem synchronen Korrelationsdiagramm.

10. Verfahren nach Anspruch 9, ferner umfassend Bestimmen des Ausmaßes der Desamidierung basierend auf der sequenziellen Abfolge der molekularen Ereignisse.

11. Verfahren nach Anspruch 8, ferner umfassend Bestimmen der Stabilität von Domänen in den Proteinen, Peptiden und/oder Peptoiden.

12. System zum Verarbeiten von Daten, die eine Eigenschaft von Proteinen, Peptiden und/oder Peptoiden darstellen, wobei das System umfasst:

ein Datenerfassungsmodul, konfiguriert, Spektraldaten zu erfassen, aufgenommen unter Verwendung eines Quantenkaskadenlaser-Mikroskops, der Proteine, Peptide und/oder Peptoide ohne Verwendung von Sonden oder Additiven in Bezug auf eine angewendete Störung; und
ein Korrelationsanalysemodul, konfiguriert zum:

Anwenden einer zweidimensionalen Korrelationsanalyse, um ein asynchrones Korrelationsdiagramm für die Proteine, Peptide und/oder Peptoide zu erzeugen; und
Identifizieren in dem asynchronen Korrelationsdiagramm mindestens eines mit Desamidierung der Proteine, Peptide und/oder Peptoide assoziierten Peaks .

13. System nach Anspruch 12, wobei das Korrelationsanalysemodul dazu eingerichtet ist:
den mindestens einen Peak zu benutzen, um eine Reihenfolge einer verteilten Präsenz von spektralen Intensitätsänderungen in Bezug auf die angewandte Störung zu bestimmen.

14. System nach Anspruch 13, wobei das Benutzen des mindestens einen Peaks umfasst:
Bestimmen, für zwei Wellenzahlen $\nu_1$ und $\nu_2$, ob der mindestens eine Peak, der den zwei Wellenzahlen entspricht, einen positiven Wert hat.

15. System nach Anspruch 13, wobei das Benutzen des mindestens einen Peaks umfasst:
Bestimmen, für zwei Wellenzahlen $\nu_1$ und $\nu_2$, ob der mindestens eine Peak, der den zwei Wellenzahlen entspricht, einen negativen Wert hat.

16. System nach Anspruch 12, wobei das Erfassen der Spektraldaten Analysieren der Seitenkettenmoden der Proteine, Peptide und/oder Peptoide als interne Sonden einschließt.

17. System nach Anspruch 12, wobei das Korrelationsanalysemodul konfiguriert ist zum:
Anwenden einer zweidimensionalen Korrelationsanalyse, um ein synchrones Korrelationsdiagramm für die Proteine, Peptide und/oder Peptoide zu erzeugen.

18. System nach Anspruch 17, wobei das Korrelationsanalysemodul ferner konfiguriert ist zum:

Bestimmen einer sequenziellen Abfolge molekularer Ereignisse aus dem asynchronen Korrelationsdiagramm und dem synchronen Korrelationsdiagramm; und

Bestimmen des Ausmaßes der Desamidierung basierend auf der sequenziellen Abfolge der molekularen Ereignisse.

19. Nichttransitorisches computerlesbares Medium, umfassend Anweisungen, die, wenn von einem oder mehreren Computern ausgeführt, die einen oder mehreren Computer dazu veranlassen:

Spektraldaten, aufgenommen unter Verwendung eines Quantenkaskadenlaser-Mikroskops, der Proteine, Peptide und/oder Peptoide, ohne Verwendung von Sonden oder Additiven in Bezug auf eine angewendete Störung zu erfassen;

Anwenden einer zweidimensionalen Korrelationsanalyse, um ein asynchrones Korrelationsdiagramm für die Proteine, Peptide und/oder Peptoide zu erzeugen; und

Identifizieren in dem asynchronen Korrelationsdiagramm mindestens eines Peaks assoziiert mit Desamidierung der Proteine, Peptide und/oder Peptoide.

**Revendications**

1. Procédé informatisé pour traiter des données représentant une caractéristique de protéines, peptides et/ou peptoïdes, le procédé comprenant :

l'obtention de données spectrales, prises au moyen d'un microscope laser à cascade quantique, des protéines, peptides et/ou peptoïdes sans utilisation de sondes ou d'additifs à l'égard d'une perturbation appliquée ;

l'application d'une analyse de corrélation bidimensionnelle pour générer un tracé de corrélation asynchrone pour les protéines, peptides et/ou peptoïdes ; et

l'identification, dans le tracé de corrélation asynchrone, d'au moins un pic associé à une désamidation des protéines, peptides et/ou peptoïdes.

2. Procédé selon la revendication 1, comprenant en outre l'utilisation de l'au moins un pic pour déterminer un ordre d'une présence distribuée de changements d'intensité spectrale à l'égard de la perturbation appliquée.

3. Procédé selon la revendication 2, dans lequel l'utilisation de l'au moins un pic comprend :
la détermination, pour deux nombres d'ondes $v_1$ et $v_2$, que l'au moins un pic correspondant aux deux nombres d'ondes a ou non une valeur positive.

4. Procédé selon la revendication 2, dans lequel l'utilisation de l'au moins un pic comprend :
la détermination, pour deux nombres d'ondes $v_1$ et $v_2$, que l'au moins un pic correspondant aux deux nombres d'ondes a ou non une valeur négative.

5. Procédé selon la revendication 1, comprenant en outre l'identification d'une pluralité de pics dans le tracé de corrélation asynchrone, et la détermination qu'un événement de désamidation est survenu quand il y a une corrélation de pics qui présentent des changements d'intensité déphasés.

6. Procédé selon la revendication 1, dans lequel l'obtention des données spectrales comprend l'analyse de modes de chaîne latérale des protéines, peptides et/ou peptoïdes servant de sondes internes.

7. Procédé selon la revendication 1, comprenant en outre l'exécution d'une analyse de codistribution bidimensionnelle sur les données spectrales.

8. Procédé selon la revendication 1, comprenant en outre :
l'application d'une analyse de corrélation bidimensionnelle pour générer un tracé de corrélation synchrone pour les protéines, peptides et/ou peptoïdes.

9. Procédé selon la revendication 8, comprenant en outre la détermination d'un ordre séquentiel d'événements moléculaires à partir du tracé de corrélation asynchrone et du tracé de corrélation synchrone.

**10.** Procédé selon la revendication 9, comprenant en outre la détermination de l'ampleur de désamidation sur la base de l'ordre séquentiel d'événements moléculaires.

**11.** Procédé selon la revendication 8, comprenant en outre la détermination de la stabilité de domaines dans les protéines, peptides et/ou peptoïdes.

**12.** Système pour traiter des données représentant une caractéristique de protéines, peptides et/ou peptoïdes, le système comprenant :

un module d'acquisition de données configuré pour obtenir des données spectrales, prises au moyen d'un microscope laser à cascade quantique, des protéines, peptides et/ou peptoïdes sans utilisation de sondes ou d'additifs à l'égard d'une perturbation appliquée ; et
un module d'analyse de corrélation configuré pour :

appliquer une analyse de corrélation bidimensionnelle pour générer un tracé de corrélation asynchrone pour les protéines, peptides et/ou peptoïdes ; et
identifier le tracé de corrélation asynchrone au niveau d'au moins un pic associé à une désamidation des protéines, peptides et/ou peptoïdes.

**13.** Système selon la revendication 12, dans lequel le module d'analyse de corrélation est configuré pour :
utiliser l'au moins un pic pour déterminer un ordre d'une présence distribuée de changements d'intensité spectrale à l'égard de la perturbation appliquée.

**14.** Système selon la revendication 13, dans lequel l'utilisation de l'au moins un pic comprend :
la détermination, pour deux nombres d'ondes $v_1$ et $v_2$, que l'au moins un pic correspondant aux deux nombres d'ondes a ou non une valeur positive.

**15.** Système selon la revendication 13, dans lequel l'utilisation de l'au moins un pic comprend :
la détermination, pour deux nombres d'ondes $v_1$ et $v_2$, que l'au moins un pic correspondant aux deux nombres d'ondes a ou non une valeur négative.

**16.** Système selon la revendication 12, dans lequel l'obtention des données spectrales comprend l'analyse de modes de chaîne latérale des protéines, peptides et/ou peptoïdes servant de sondes internes.

**17.** Système selon la revendication 12, dans lequel le module d'analyse de corrélation est configuré pour :
appliquer une analyse de corrélation bidimensionnelle pour générer un tracé de corrélation synchrone pour les protéines, peptides et/ou peptoïdes.

**18.** Système selon la revendication 17, dans lequel le module d'analyse de corrélation est en outre configuré pour :

déterminer un ordre séquentiel d'événements moléculaires à partir du tracé de corrélation asynchrone et du tracé de corrélation synchrone ; et
déterminer l'ampleur de désamidation sur la base de l'ordre séquentiel d'événements moléculaires.

**19.** Support lisible sur ordinateur non transitoire comprenant des instructions qui, lorsqu'elles sont exécutées par un ou plusieurs ordinateurs, conduisent le ou les ordinateurs à :

obtenir des données spectrales, prises au moyen d'un microscope laser à cascade quantique, des protéines, peptides et/ou peptoïdes sans utilisation de sondes ou d'additifs à l'égard d'une perturbation appliquée ;
appliquer une analyse de corrélation bidimensionnelle pour générer un tracé de corrélation asynchrone pour les protéines, peptides et/ou peptoïdes ; et
identifier, dans le tracé de corrélation asynchrone, au moins un pic associé à une désamidation des protéines, peptides et/ou peptoïdes.

**FIG. 1**

FIG. 2A

(A) → Load experimental data → Calculate signal to noise ratio (S/N) → S/N > threshold ?

S/N > threshold ? — **No** → Display error → Failure

S/N > threshold ? — **Yes** → Apply baseline correction → Normal distribution analysis → Intensity FOV same ?

Intensity FOV same ? — **No** → Calculate aggregate size → Select ROI's →

Intensity FOV same ? — **Yes** → Calculate mean → Calculate covariance → Calculate correlation → Success

Calculate mean → ⊕ → Calculate Co-distribution → Calculate correlation → Success

**FIG. 2B**

**310:** Raw Spectra

Absorbanc[e]

Wavenumber (cm⁻¹)

Temperature

28.0
32.0
36.0
40.0
44.0

ΔI

**320.** Covariance Spectra

Wavenumber (cm⁻¹)

**330.** 2D IR Correlation Algorithm

**370.** Co-Distribution Correlation Algorithm

$v_1$ $v_2$

Population Distribution Dynamics

Correlation of In-Phase Peak Changes

**340.** Synchronous Plot

$v$ $v_2$

$v_1$ $v_2$

**350.** Asynchronous Plot

Correlation of Out-of-Phase Peak Changes

Description of Molecular Dynamics

**360.** Sequential Order of Events

**FIG. 3**

EP 3 870 193 B1

FIG. 4A

FIG. 4B

T=56 °C

T=28 °C

FIG. 4C

FIG. 5

EP 3 870 193 B1

FIG. 6A

FIG. 6B

FIG. 6C

**FIG. 7A**

NIST mAb

Absorbance

Wavenumber (cm$^{-1}$)

**FIG. 7B**

1e-3

2.70 — 2.25 — 1.80 — 1.35 — 0.90 — 0.45 — 0.00 — -0.45

Wavenumber cm$^{-1}$, $v_1$ vs Wavenumber cm$^{-1}$, $v_2$

**FIG. 7C**

1e-5

9.6 — 7.2 — 4.8 — 2.4 — 0.0 — -2.4 — -4.8 — -7.2 — -9.6

Wavenumber cm$^{-1}$, $v_1$ vs Wavenumber cm$^{-1}$, $v_2$

EP 3 870 193 B1

FIG. 8A — "NIST mAb Candidate", Absorbance vs Wavenumber (cm⁻¹)

FIG. 8B — Wavenumber cm⁻¹, $\nu_1$ vs Wavenumber cm⁻¹, $\nu_2$ (scale 1e-3)

FIG. 8C — Wavenumber cm⁻¹, $\nu_1$ vs Wavenumber cm⁻¹, $\nu_2$ (scale 1e-4)

Sequential Order of Molecular Events

FIG. 9A

Sequential Order of Molecular Events

FIG. 9B

**NIST mAb Candidate (RM 8670) at 1.5 μg/μL**

Temperature

Stability

1519.0
Tyr \ 1525.0
Lys \ 1540.7
δ$_s$(NH$_3$) Glu⁻ \ 1580.0
ν(COO-) Asp⁻ \ 1591.2
ν(COO-) Gln \ 1621.0
δ(NH$_2$) Lys \ 1612.7
δ$_s$(NH$_3$) Asn \ 1600.1
δ(NH$_2$) His \ 1635.6
β-sheet \ 1653.8
α-helix \ 1665.2
hinge \ 1670.3
loop Gln \ 1559.0
ν(C=O) Glu⁻ \ 1572.0
ν(COO-) Asp⁻ \ 1678.3
ν(COO-) Asn \ 1726.7
ν(C=O) Phe/Tyr \ 1748.7
p-Ar Phe/Tyr \ 1705.0
p-Ar Phe/Tyr \ 1693.1
p-Ar β-turn

**Sequential Order of Molecular Events**

**FIG. 9C**

EP 3 870 193 B1

FIG. 10A

FIG. 10B

Wavenumber cm$^{-1}$, v$_1$ vs Wavenumber cm$^{-1}$, v$_2$

FIG. 10C

Wavenumber cm$^{-1}$, v$_1$ vs Wavenumber cm$^{-1}$, v$_2$

FIG. 11A

FIG. 11B

FIG. 11C

FIG. 12B

Wavenumber cm$^{-1}$, v$_1$ vs Wavenumber cm$^{-1}$, v$_2$

FIG. 12C

Wavenumber cm$^{-1}$, v$_1$ vs Wavenumber cm$^{-1}$, v$_2$

NIST mAb Cadidate

FIG. 12A

Wavenumber (cm$^{-1}$)

Absorbance

Sequential Order of Molecular Events

FIG. 13A

EP 3 870 193 B1

Sequential Order of Molecular Events

FIG. 13B

EP 3 870 193 B1

**NIST mAb Candidate (RM 8670) at 2.4 μg/μL**

1519.0
Tyr   \1525.0
Lys   \1540.7
$\delta_s(NH_3)$   Glu⁻   \1635.6
v(COO-)   β-sheet   \1653.8
α-helix   \1580.0
Asp   \1621.0
v(COO-)   Lys   \1612.7
$\delta_{as}(NH_3)$   **Asn**   \1665.2
$\delta(NH_2)$   hinge   \1600.1
loop   His   \1591.2
**Gln**   \1670.0
$\delta(NH_2)$   **Gln**   \1559.0
v(C=O)   Glu⁻   \1572.0
v(COO-)   **Asp⁻**   \1678.3
v(COO-)   Asn   \1726.7
v(C=O)   Phe/Tyr   \1748.7
p-Ar   Phe/Tyr   \1705.0
p-Ar   Phe/Tyr   \1693.1
p-Ar   β-turn

**Sequential Order of Molecular Events**

**FIG. 13C**

FIG. 14A

FIG. 14B

FIG. 14C

NIST mAb Cadidate

**FIG. 15A**

Wavenumber cm$^{-1}$, v$_1$ vs Wavenumber cm$^{-1}$, v$_2$

**FIG. 15B**

Wavenumber cm$^{-1}$, v$_1$ vs Wavenumber cm$^{-1}$, v$_2$

**FIG. 15C**

Sequential Order of Molecular Events

FIG. 16A

**NIST mAb Candidate (RM 8670) at 10 μg/μL**

1519.0 / Tyr

\1525.0 / Lys / δ_s(NH_3)

\1540.7 / Glu⁻ / ν(COO-)

\1559.0 / Glu⁻ / ν(COO-)

\1580.2 / Asp⁻ / ν(COO-)

\1572.0 / Asp⁻ / ν(COO-)

\1635.6 / β-sheet

\1653.8 / α-helix

\1665.2 / hinge loop

\1670.0 / Gln / ν(C=O)

\1621.0 / Lys / δ_as(NH_3)

\1612.7 / Asn / δ(NH_2)

\1591.0 / Gln / δ(NH_2)

\1598.9 / His

\1678.3 / Asn / ν(C=O)

\1726.7 / Phe/Tyr / p-Ar

\1748.7 / Phe/Tyr / p-Ar

\1705.0 / Phe/Tyr / p-Ar

\1693.1 / β-turn

Temperature

Stability

**Sequential Order of Molecular Events**

**FIG. 16B**

FIG. 17B

NIST mAb

Wavenumber cm$^{-1}$, $v_1$ vs Wavenumber cm$^{-1}$, $v_2$

FIG. 17C

NIST mAb Candidate

Wavenumber cm$^{-1}$, $v_1$ vs Wavenumber cm$^{-1}$, $v_2$

FIG. 17A

PDS NIST mAb

Wavenumber cm$^{-1}$, $v_1$ vs Wavenumber cm$^{-1}$, $v_2$

**PDS NIST mAb (RM 8671)**  **NIST mAb (RM 8671)**  **NIST mAb Candidate (RM 8670)**

FIG. 18A          FIG. 18B          FIG. 18C

EP 3 870 193 B1

**Cross Peak Intensity Changes**

NIST mAb Candidate

1635,1559

a

b

1635,1591

c

1635,1670

α/β

1653,1635

$\Delta I$

4.0x10$^{-5}$
2.0x10$^{-5}$
0.0
-2.0x10$^{-5}$
-4.0x10$^{-5}$
-6.0x10$^{-5}$
-8.0x10$^{-5}$

**FIG. 20**

**2D IR Asynchronous Plot**

1e-4

1.2
0.9
0.6
0.3
0.0
-0.3
-0.6
-0.9
-1.2

1500  1550  1600  1650  1700  1750

1500  1550  1600  1650  1700  1750

Wavenumber cm$^{-1}$, $v_1$ vs Wavenumber cm$^{-1}$, $v_2$

**FIG. 19**

FIG. 21

FIG. 22

**2301**
**Design of Experiment (DOE)**

**2302**
**Correlation Dynamics Software**

**2303**
**Comparative Analysis**

FIG. 23

Stepwise approach to assessing immunogenicity of biopharmaceuticals. *ADA* anti-durg antibody

**Direct method of detection**
May provide human sera evaluation as a result aid in the design of the clinical trial.

**May have similar sensitivity than current Bioassay**
Resulting in inclusion as part of the IND and/or BLA filing.

**Increase Safety and Efficacy**
May be a predict solution resulting in decreasing the risk of immunogenicity or ADA

**FIG. 24**

## Comparative Analysis
## Immunogenicity Risk Assessment

| Screen Bioassay | Platform Technology |
| --- | --- |
| Assay Result | Direct Measurement |

Normal Distribution Analysis
prob < W

Normal Distribution Analysis
prob < W

Negative

N/A

Positive → Negative

→ Positive     Aggregation Evaluation

## ADA Risk Assessment

| Screen Bioassay | Platform Technology |
| --- | --- |
| Assay Result | Direct Measurement |

Normal Distribution Analysis
prob < W

Normal Distribution Analysis
prob < W

Negative

N/A

Positive → Negative

→ Positive     Aggregation Evaluation

Positive     Assess ADA

## FIG. 25

1902 Processor

1904 Memory

1906 Data Storage

1908 Bus

Input/Output Module
1910

Device
1914

Device
1916

**FIG. 26**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2017014338 W

**Non-patent literature cited in the description**

• Recent advancements, challenges, and practical considerations in the mass spectrometry-based analytics of protein biotherapeutics: A viewpoint from the biosimilar industry. **HÁDA VIKTOR et al.** JOURNAL OF PHARMACEUTICAL AND BIOMEDICAL ANALYSIS. ELSEVIER B.V, 11 August 2018, vol. 161, 214-238

• **ISAO NODA**. Two-dimensional co-distribution spectroscopy to determine the sequential order of distributed presence of species. *Journal of Molecular Structure*, vol. 1069, 51-54
• **ISAO NODA**. Two-dimensional co-distribution spectroscopy to determine the sequential order of distributed presence of species. *Journal of Molecular Structure*, vol. 1069, 54-56